# EUROPEAN PATENT SPECIFICATION

(11) **EP 1 648 520 B1**
(45) Date of publication and mention of the grant of the patent: **05.05.2010**
(21) Application number: 04737968.0
(22) Date of filing: 15.07.2004
(51) Int. Cl.: A61K 48/00, A61K 38/00, C07K 14/47

(54) **COMPOUNDS AND METHODS FOR DOWNREGULATING THE EFFECTS OF TGF-BETA**
VERBINDUNGEN UND METHODEN FÜR DAS DOWNREGULATING DER EFFEKTE VON TGF-BETA
COMPOSES ET METHODES PERMETTANT UNE REGULATION NEGATIVE DES EFFETS DU TGF-BETA

(30) Priority: 16.07.2003 US 487659 P
(43) Date of publication of application: 26.04.2006
(73) Proprietor: RVX Therapeutics, Inc., Calgary AB T2X 1M2 (CA)
(72) Inventor: MIHARA, Koichiro, Calgary, Alberta T2N 4B6 (CA); WONG, Norman, C. W., Calgary, Alberta T3A 2K8 (CA); LEBIODA, Kenneth, Calgary, Alberta T3Z 3T2 (CA); TUCKER, Joe, Calgary, Alberta T3G 4Z2 (CA)
(74) Representative: Vossius & Partner
(86) International application number: PCT/CA2004/001036
(87) International publication number: WO 2005/007199

(56) References cited:
- EP-A- 1 160 319
- WO-A-02/39923
- WO-A-02/094201
- DEMETRIOU M ET AL: "FETUIN/ALPHA2-HS GLYCOPROTEIN IS A TRANSFORMING GROWTH FACTOR-BETA TYPE II RECEPTOR MIMIC AND CYTOKINE ANTAGONIST" JOURNAL OF BIOLOGICAL CHEMISTRY, AMERICAN SOCIETY OF BIOLOGICAL CHEMISTS, BALTIMORE, MD, US, vol. 271, no. 22, 31 May 1996 (1996-05-31), pages 12755-12761, XP002906747 ISSN: 0021-9258
- PARTRIDGE EMILY ET AL: "alpha2-HS-glycoprotein (Ahsg)/fetuin inhibits TGF-beta1 signaling in cancer cells" PROCEEDINGS OF THE AMERICAN ASSOCIATION FOR CANCER RESEARCH ANNUAL MEETING, vol. 43, March 2002 (2002-03), page 20, XP001183663 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- TAJIRIAN TANIA ET AL: "Regulation of human monocyte proMMP-9 production by fetuin, an endogenous TGF-beta antagonist" JOURNAL OF CELLULAR PHYSIOLOGY, vol. 185, no. 2, November 2000 (2000-11), pages 174-183, XP009037261 ISSN: 0021-9541
- GORELIK LEONID ET AL: "Immune-mediated eradication of tumors through the blockade of transforming growth factor-beta signaling in T cells" NATURE MEDICINE, vol. 7, no. 10, October 2001 (2001-10), pages 1118-1122, XP002298291 ISSN: 1078-8956
- SHAH ALI H ET AL: "Suppression of tumor metastasis by blockade of transforming growth factor beta signaling in bone marrow cells through a retroviral-mediated gene therapy in mice." CANCER RESEARCH, vol. 62, no. 24, 15 December 2002 (2002-12-15), pages 7135-7138, XP002298292 ISSN: 0008-5472
- WU R S ET AL: "COMPARATIVE ANALYSIS IFN-GAMMA B7.1 AND ANTISENSE TGF-BETA GENE TRANSFER ON THE TUMORIGENICITY OF A POORLY IMMUNOGENIC METASTATIC MAMMARY CARCINOMA" CANCER IMMUNOLOGY AND IMMUNOTHERAPY, BERLIN, DE, vol. 50, no. 5, 2001, pages 229-240, XP001038551 ISSN: 0340-7004
- TZAI T-S ET AL: "ANTISENSE OLIGONUCLEOTIDE SPECIFIC FOR TRANSFORMING GROWTH FACTOR-BETA1 INHIBIT BOTH IN VITRO AND IN VIVO GROWTH OF MBT-2 MURINE BLADDER CANCER" ANTICANCER RESEARCH, HELENIC ANTICANCER INSTITUTE, ATHENS,, GR, vol. 18, no. 3A, May 1998 (1998-05), pages 1585-1589, XP001038615 ISSN: 0250-7005

## Description

### Related Applications

This application claims the benefit of U.S. Provisional Application No. 60/487,659 filed July 16, 2003.

### Field of the Invention

The present invention relates to the field of enhancing immunological response to cancer and cellular therapeutic methods for treating cancer and in particular to compounds, such as AHSG, and methods for downregulating the biological effects of TGF-β.

### Background of the Invention

Transforming Growth Factor-β ("TGF-β") is a member of a dimeric polypeptide growth factor family and plays an essential role in signaling pathways that regulate proliferation and differentiation of cells, embryonic development, wound healing and angiogenesis. Almost every cell in the body produces TGF-β and has receptors that bind the growth factor. The actions of TGF-β are implicated in many disease states, including atherosclerosis; fibrotic diseases of the lung, liver, kidney and cardiovascular system; excessive wound healing; and cancer.

### (a) Eye

TGF-β is the most potent growth factor in the body for promoting wound healing. Numerous disorders and diseases of the eye involve a progression to blindness that results from a scarring response to a lesion. This natural scarring response is mediated by TGF-β and is the target of several therapeutic strategies that have thus far proved ineffective.

Examples of disorders in which scarring, caused by TGF-β induced wound healing leading to loss or reduction of vision, include scarring following glaucoma surgery, as well as corneal, cataract and posterior capsular scarring. Furthermore, TGF-β plays a role in proliferative vitreoretinopathy, macular degeneration and other proliferative diseases of the eye.

Proliferative vitreoretinopathy is a disease process that occurs in 1 out of 10 eyes surgically treated for retinal detachment; and the disease may lead to blindness. In this disorder, excessive intraocular fibrosis is linked to a significant increase in TGF-β activity, suggesting that an effective strategy to reduce intraocular TGF-β levels may limit or halt the progression of the disease.

Approaches to inhibit TGF-β activity in the eye as a therapeutic modality have met with little success due to a variety of problems. For approaches targeting proliferative diseases, antiproliferative drugs such as those used in cancer therapy (e.g. mytomycin and fluorouracil) are used, but with significant limitation of utility due to the drugs' toxic side effects of leaking blebs leading to blinding inflections and hypotony. Approaches to specifically target factors involved in wound healing have thus far been unsuccessful in garnering regulatory approval. An antibody specific for TGF-β, lerdelimumab, has been developed and has to date reached late stage human clinical testing for optic disorders. Delivery of an antibody to the eye is restricted in its usefulness as a therapy because of its limited time of retention in the tissue, requiring repeated applications; and also because of the possibility that immunogenicity and inappropriate targeting could be mediated by the antibody itself. Antisense oligonucleotides targeting TGF-β activity (in vivo) are also in development for the treatment of disorders of postoperative scarring of the eye, but these agents face numerous challenges, including those common to all antisense approaches, such as nuclease stability, antisense potency and targeting specificity, among others.

It is an aspect of the present invention to provide improved therapeutic agents, and methods for selecting same, for treating disorders of the eye including especially those mediated by TGF-β.

### (b) Liver

TGF-β is a key cytokine involved in the modulation of liver fibrogenesis, both in human disorders such as toxic, cholestatic, alcoholic (e.g. cirrhosis), inflammatory and other type of liver injury and also in animal models of these disorders. Increases in TGF-β activity play an important role in the development and progression of these disorders, and thus reducing TGF-β activity is an attractive therapeutic target

The only curative treatment for end stage liver fibrosis is transplantation, but donor numbers and the clinical condition of the patient limit this therapeutic approach. Other therapies attempt to relieve the symptoms of fibrosis, or to block the underlying cause for the damage to the liver that has resulted in the fibroproliferative pathology. However, no therapies to date directly treat liver fibrosis.

It is a further aspect of the present invention to provide an improved therapeutic approach for the treatment of liver fibrosis.

### (c) Kidney

Fibrotic disease in the kidney bears much resemblance to that in the liver, it is specifically untreatable with much of the progression of the disease mediated by the activities of TGF-β. Angiotensin-converting enzyme (ACE) inhibitors may delay the progression of diabetic kidney disease, and angiotensin receptor blockers (ARBs) have shown benefit in slowing the loss of renal function in Type-II diabetic nephropathy. The effects of both drug classes are believed to derive primarily from the relaxation of efferent arteriolar constriction and the release of intraglomerular pressure that they mediate, but may also in part be caused by the reduction of TGF-β activating cytokine activity of angiotensin that both classes of drugs also mediate. However, both ACE-inhibitors and ARBs are unable to completely normalize the levels of TGF-β in the pathological kidney and also are unable to prevent or delay end-stage renal disease in all patients.

It is a further aspect of the present invention to provide an improved therapeutic approach for the treatment of kidney diseases, especially those medicated by TGF-β.

### (d) Pulmonary fibrotic diseases

Pulmonary fibrosis is characterized by the enhanced synthesis and deposition of extracellular matrix in the lungs; with the pathogenesis of this disease driven, in large part, by increased TGF-β activity. Standard treatments for idiopathic pulmonary fibrosis are steroids and immunosuppressive agents. As the 5-year survival rate for this disease is less than 50%, the current therapies are clearly inadequate. No therapeutics are currently available for the treatment of pulmonary fibrosis that directly inhibit or downregulate TGF-β activity to halt or reverse the progression of the disease.

It is a still further aspect of the present invention to provide an improved therapeutic approach for the treatment of pulmonary fibrosis.

### (e) Myocardial fibrosis

Diastolic dysfunction is a common characteristic of hypertensive heart disease and cardiomyopathy. Excessive deposition of fibrous tissue is a major cause of reduced pumping capacity in hypertrophied hearts and of a number of other ventricular dysfunctions. Unfortunately, no specific therapy is yet available to improve left ventricular diastolic function and it is accordingly yet another aspect of the present invention to provide such a method.

TGF-β is the primary cytokine in the heart controlling the proliferation of fibroblasts and subsequent deposition of extracellular matrix that composes, in large part, the fibrous tissue mass which underlies the disease symptoms. Experiments in animals have demonstrated a causal link between TGF-β overexpression and pathogenic fibrosis in pressure overloaded hearts. Furthermore, human studies have confirmed the presence of increased TGF-β expression in cardiac hypertrophy and fibrosis. The use of anti-TGF-β monoclonal antibodies has been proposed but not yet demonstrated to be effective in human therapy; and in any event, will be limited by the restricted duration that an injected antibody protein will remain in the body at the necessary site for therapeutic effectiveness in an undegraded statue.

It is another further aspect of the present invention to provide a therapy for diastolic dysfunction.

It is another still further aspect of the present invention to address myocardial fibrosis with a more focused therapeutic approach.

### (f) Gene Therapy

Gene therapy approaches seek to ameliorate diseases by transferring to a target tissue, genes that encode beneficial products, antisense DNA or RNA, RNA-decoys or ribozymes. In general, gene therapy approaches have the potential to overcome obstacles facing the targeted delivery of proteins and RNA and also to improve efficacy while providing a longer duration of effect and even potentially greater safety. In animals gene therapy has been shown to be effective in models such as rheumatoid arthritis, multiple sclerosis, diabetes and lupus erythematodus. Over 400 human clinical studies have been or are in the process of being performed, but no gene therapy approach has yet been approved for any of the fibrotic disorders mentioned herein.

It is still another aspect of the present invention to provide therapeutic agents which capitalize on gene therapy approaches.

Despite the immunogenicity associated with tumor cells, they still have the uncanny ability to hide from the body's surveillance system; with both cytotoxic lymphocytes ("CTL") and natural killer ("NK") cells being key components of immune surveillance.

It is yet still another aspect of the present invention to provide methods to overcome the cloaking mechanism(s) that are believed to be inherent features of certain cancer cells.

It is still yet another further aspect of the present invention to enhance the function of cytotoxic lymphocyte and natural killer cells to present an attractive method for cancer therapy.

It is a further aspect of the present invention to provide a novel cDNA suitable for transducing or transfor-ming lymphocytes to express AHSG whereby such protein provides a bifunctional role through competitive binding to TGF-β receptors, thereby antagonizing its actions, and acting as a potent inhibitor of cathepsin B activity.

A more conventional approach has relied on enhancing the immunogenicity of tumor cells. For example, studies have attempted to enable tumor cells to express cytokines such as interferons (IL-2, IL-12) and granulocyte/macrophage colony stimulating factor ("GM-CSF"). The expression of any one of these proteins enhances the ability of the immune system to detect the cancer cells (Smith, I.J. eft al J Immunother 26:130(2003)). The expression of any one of these genes may lead to the regression or retardation of the growth of cancer cells *in vitro* (Rosenberg, S.A. Nature 411:380(2001)). Unfortunately, although the interferons work well *in vitro*, the results of human clinical trials have been disappointing and the predicted success from the *in vitro* studies has not materialized (de Visser, K. E. et al Leukemia 13:1188 (1999); Wojtowicz - Praga, S. J. Immunother 20:165(1997)).

Another way to render tumor cells more susceptible to the immune system involves the expression of so called accessory molecules such as B7-1 and B7-2. The insertion of either gene into cancer cells induces expression of the major histocompatibility complex ("MHC"). The presence of this complex on tumor cells facilitates recognition of the target cells by lymphocytes (de Visser, K. E. et al Leukemia 13:1188 (1999); Wojtowicz - Praga, S. J. Immunother 20:165(1997). Another potential avenue of treatment research relies on the features of NK cells which are part of the innate immune system. Information arising from these studies will allow development of new ways to activate NK cells in the future (Colucci, F. et al Nat Rev Immunol 3:413 (2003)). Previous efforts to augment immune activity were often accompanied by an inflammation reaction, such as the studies using IL-2.

Factor(s) that suppress the function of immune cells include, for example, TGF-β; a classic example of a growth factor that suppresses function of the immune cells. Cancer cells that express TGF-β use this protein to suppress the function of selected cells of immune surveillance and thus escape from being detected (Colucci, F. et al Nat Rev Immunol 3:413 (2003)). Many studies have and continue to document the relationship of TGF-β mRNA in various cancer cells; or the presence of the growth factor in serum from patients with malignancies including glioma, melanoma, breast, colon, gastric and prostate cancers Teicher, B.A. Cancer Metastasis Rev. 20:133 (2001); Wieser, R. Corr Opin Oncol 13:70 (2001); Pasche, B. J Cell Physiol 186:153 (2001)).

The mechanisms by which TGF-β action lends to the malignant potential of cancers have been reviewed (Wojtowicz-Praga, S. J Immunother 20:165 (1997); de Visser, K.E. et al Leukemia 13:1188 (1999); Derynck, R. et al. Nat Genet 29:117 (2001). An elegant demonstration of the importance of TGF-β in tumorogenesis comes from studies of knock-out mice where expression of the Type-II TGF-β receptor was not evident in T-cell lymphocytes. Cells taken from these mice have the ability to eradicate TGF-β expressing tumors in a syngenic mouse model (Gorelik, L. et al Nature Rev Immunol 2:46 (2002)).

The features of TGF-β are reviewed in an article by Sporn (Sporn, M.B. Microbes Infect 1:1251 (1999)) and the signal transduction activity of the factor is detailed in other reviews (Roberts, A.B. Microbes Infect 1:1265 (1999); Massague, J. Nat Rev Mol Cell Biol 1:169 (2000)). In brief, TGF-β is secreted from cells in a latent form and serum contains significant levels of the latent form of the factor. Latent TGF-β does not bind to TGF-β receptors. A110 kD homodimer of latency associated protein ("LAP") is a part of this TGF-β precursor. LAP binds to one of at least four different specific carrier proteins, called large TGF-β binding proteins ("LABP"). LTBP are involved in activation and storage of the latent TGF-β. Active TGF-β is a 25 kD homodimer that is cleaved from the latent form of TGF-β. The latent form of TGF-β circulates in the serum and it can be activated by several proteases. One group of these is the matrix metalloproteinases ("MMP") that can activate the latent form of TGF-β. Activated TGF-β suppresses expression' of MMP inhibitors, for example tissue inhibitor of metalloproteinases ("TIMP"). The participation of these proteases in activating TGF-β is associated with augmenting the invasive ability of tumor cells (Stamenkovic, I. Semin cancer Biol 10:415 (2000)). Inhibition of the protease mediated activation mechanism may also serve as a potential target to block TGF-β action.

The binding of TGF-β to Type-I and Type-II receptors on the cell surface serve as the first step in mediating signal transduction initiated by the growth factor. TGF-β elicits its effects by binding to a heteromeric complex of transmembrane Serine/Threonine kinase receptors comprised of both Type-I and Type-II isoforms. The TGF-β Type-II receptor ("TbR-II") binds directly to TGF-β, followed by recruitment and phosphorylation of the TGF-β Type-I receptor ("ThR-I"), which in turn triggers the signal transduction pathways. Consequently, agents that mimic or suppress TbR-II provide another promising avenue to antagonize the action of TGF-β.

Others have attempted to stimulate tumor immunity using TGF-β antagonists, such as the TGF-β binding protein, decorin (Monz, C. et al Eur J Immunol 29:1032 (1999); Stander, M. et at Gene Ther 5:1187 (1998)).; or a chimeric molecule of extra-cellular domain TbR-II fused to the F_{c} region of human immunoglobulin with limited success (Won J. et al Cancer Res 59:1273 (1999); Komesli, S. et al Eur J Biochem 254:505 (1998)). Others showed that α₂-macroglobulin protein also has the ability to antagonize the action of TGF-β (Harthun, N.L. et al J Immunother 21:85 (1998)). However, this protein is difficult to use because of its large mass (720kD) and the protein exists as a tetramer. Another study suggests that a 20 amino acid peptide fragment of the protein has TGF-β binding activity (Webb, D.J. et al Protein Sci 9:1986 (2000)).

α2-Heremans-Schmidglycoprotein ("AHSG") is a globulin like protein and is the human homolog of bovine protein, fetuin. Fetuin was identified as a major protein component of fetal strum (Pedersen, K. Nature 154:570 (1944)). The function of fetuin may be related to the development and maturation of the hematopoietic and immune systems (Dziegielewska, K. et al Histochem Cell Biol 106:319 (1996)). Like fetuin, AHSG is secreted from liver as a negative acute-phase protein (Lebreton J. P. et al J Clin Invest 64:1118 (1979)). The protein structure of AHSG reveals that it belongs to a member of the cystatin superfamily. A characteristic feature of this superfamily of proteins is that they possess two tandemly arranged cystatin domains and a third domain that is rich in proline and glycine. Secondary modifications of the protein include N-glycosylation, O-glycosylation (Hayase, T. et al Biochemistry 31:4915 (1992); Edge, A.S. et al J Biol Chem 262: 16135 (1987) and Serine phosphorylation (Jahnen-Dechant, W. et al Eur J. Biochem 226:59 (1994)). These modifications have been described for fetuin from a variety of species. AHSG contains two cystatin domains (Elzanowski, A. et al FEBS Lett 227:167(1988)). The cystatin domain is conserved in the superfamily of cysteine proteinase inhibitors. This domain is characterized by at least one repeat of 100-120 amino acid residues containing a domain with the conserved sequence motifs.

The mature form of human AHSG has two polypeptide chains that are connected by a disulfide bond between cystein residues. These two polypeptide chains are produced by proteolytic cleavage from a single polypeptide (Jahnen-Dechant, W. et al Eur J Biochem 226:59 (1994)). Functions proposed for AHSG are diverse: opsonization, lipid transport, cell proliferation, tyrosine kinase inhibition of the insulin receptor, protease inhibition and hematopoietic cell homing (Brown, W. M. et al Protein Sci 6:5 (1997)). A rat phosphorylated N-glycoprotein, pp63, with a mass of 63-kDa is the phosphorylated form of rat fetuin. This codified form of fetuin has been reported to bind to hepatocyte growth factor ("HGF") and thus inhibit its activity (Ohnishi, T. et al Eur J Biochem 243:753 (1997)). AHSG has also been reported to antagonize the actions of TGF-β by competitive binding to the TGF-β receptor (Demetriou, M. et al J Biol Chem 2711:12755 (1996)).

Certain tumors arise due to a dependence on TGF-β as the presence of this factor in tumor cells allows them to avoid immune system detection (Derynck, R. et al Nat Genet 29:111 (2001)). It has also been reported that lymphocytes from 'khock-out'-mice that do not express TGF-β receptor Type-II, were resistant to the actions of TGF-β and the cells of such mice had the ability to eradicate tumors in other mice⁽⁹⁾.

There are two means to antagonize the activity of TGF-β taught by the prior art. The first is to use a matrix binding type protein, an example of which is decorin (Hausser, H. et al FEBS Lett 353:243 (1994)). This particular antagonist surrounds the tumor tissue thereby reducing the concentration of active TGF-β that can reach the cell. The introduction of decorin into a malignant glioma cell line successfully reduced its malignancy potential (Stander, M. et al Gene Ther 5:1187 (1998)). The second approach of antagonizing the action of TGF-β is to use a secreted protein such as AHSG, TbRII-Fc or α2-macroglobin.

US Patent No. 5,543,143 to Read et al describes the administration of TGF-β antagonists to mammals to activate macrophages for treating infectious diseases, however there is no disclosure for using TGF-β antagonists for treating cancer. Recently, a soluble form of the TGF-β receptor and related anti-sense methods for use as TGF-β inhibitors were disclosed in US Patent Application No. 09/734,300 to Koteliansky et al; and U.S. Patent Application No. 10/146,058 to Sclingensiepen et al. The serum protein AHSG has the ability to compete with TGF-β to bind to the TGF-β receptor through a peptide domain within AHSG homologous to the consensus binding motif of TGC-β preceptor.

It is a further aspect of the present invention to increase the anti-tumor effects of TGF-β.

It is a further aspect of the present invention to utilize AHSG to compete with TGF-β in binding to TFG-β receptors.

It is a further aspect of the present invention to provide new therapeutic methods for enhancing the immunological capacity of lymphocytes to recognize and destroy cancer cells thereby providing improved methods to treating cancer.

### Summary of the Invention

The present invention is defined in the claims.

In accordance with the various aspects and principles of the present invention there are provided new ways of enabling lymphocytes to more actively combat tumor growth comprising transforming such cells with DNA capable of expressing AHSG for use in treating patients suffering from-or threatened by cancer. Such transformed cells have been discovered to have increased *in vivo* and *in vitro* activity against cancer cells. Without wishing to be bound to any particular theory or explanation, it is believed that this occurs through the effect of AHSG as an antagonist of TGF-β in the micro-environment surrounding the cancer cells.

AHSG is the human homolog of the bovine hepatic protein called fetuin. Although the function of AHSG is not fully defined, it is suspected to play a role in blocking the actions of proteins that belong to a family of proteins which includes TGF-β.

The blood concentration of AHSG in a human adult is reported to range from 0.3 to 0.6 mg/ml (Lebreton, J.P. et al J Clin Invest 64:1118 (1979)). Despite the high concentration of AHSG in the serum, this is not sufficient to block the actions of tumor cells that use TGF-β triggered mechanisms, which allow them to evade the immune response. To overcome this survival mechanism of tumor cells, we have expressed AHSG in lymphocytes and augmented the cytotoxic activity of these cells *in vivo* and *in vitro.* These results point to the importance of the microenvironment surrounding the lymphocyte and that secretion of the TGF-β antagonist, AHSG, into this environment allows it to act in an autocrine fashion and thus modify functionality of the lymphocyte.

The present invention also provides a new way to attack tumor and tumor lesions using a cell based approach. It is clear that significant levels of AHSG in the blood by itself is insufficient to block the initiation or growth of tumors, however raising local levels of the protein can suppress the activity of TGF-β at the tumor site.

As T-lymphocytes and NK cells play a critical role in the host immune response to cancer and TGF-β function as a strong suppressor of the immune response, new uses of an antagonist or inhibitor of this cytokine, such as AHSG, may be advantageously used in cancer cell therapy. The new methods provided by the present invention employ AHSG in combination with lymphoid cells from human, mouse and CD8⁺ cells or other cell lines. Without modification, exposure to TGF-β suppressed the cells' ability to incorporate ³[H]-thymidine. In contrast, cells transfected or transformed with a vector pSR-AHSG resulting in expression of AHSG, did not exhibit inhibited growth in the presence of TGF-β.

These effects were also demonstrated in another embodiment of the present invention when AHSG cDNA was cloned into an adenovirus associated vector ("AAV"). The production of AAV-AHSG viral particles from the vector was used to transform lymphoid cells. The transformed lymphocytic cells expressed AHSG mRNA and thus synthesized the AHSG protein. AAV advantageously provided a high efficiency of gene transfer and persistent expression of AHSG. Where treatment of unmodified cells with TGF-β significantly inhibited the ability of the cells to proliferate, the growth of cells infected or transformed with AAV-AHSG was not significantly different from that of untreated cells. These studies showed that TGF-β acted to suppress the growth of the wild-type non-AHSG expressing cells. In contrast, the AHSG expressing cells were guarded against the actions of TGF-β and thus the growth was not suppressed by TGF-β and grew just like the untreated cells.

The cytotoxic activity effect of cells infected with AAV-AHSG was tested against the Lewis lung carcinoma cells ("LLC1"), a malignant cell line derived from the C37BL strain of mouse. T-cell lymphocytes were isolated from the spleens of LLC1 bearing mice, cultured, amplified and infected with AAV-AHSG. Significant induction of cytotoxic activity against LLC1 cells was observed *in vitro* when contacted with lymphocyte cells infected with AAV-AHSG. *In vivo* cytotoxic activity against LLC1 was also demonstrated when AAV-AHSG infected T cell lymphocytes were injected with a mixture of LLC1 into mice. The injected mice were monitored for tumor growth. In mice injected with LLC1 and lymphocytes carrying AHSG, 60% of the mice were protected against tumor growth. This action was specific and was not seen in other animals carrying B16-F1 tumors. Thus lymphocytes isolated from LLC1 bearing mice possess the ability to seek out the cancer, but not the ability to kill it because the cancer cells secrete TGF-β to hide from the lymphocytes. Lymphocytes isolated from LLC1 tumor bearing mice were modified with AAV-AHSG to equip them with the ability to express a TGF-β antagonist. Such modified cells not only have the ability to seek out cancer cells, but can also destroy them because of their ability to antagonize or overcome the protective effects of TGF-β expressed by the cancer cells.

Two different techniques were employed to test the ability of the modified or unmodified lymphocytes to kill LLC cells. In the first, an *in vitro* mixing study was done where LLC1 and lymphocytes were mixed and then injected. In mice injected with a mixture of LLC1 plus TGF-β expressing total lymphocytes, 60% were tumor free. In contrast, none or 0% of mice injected with LLC1 and unmodified lymphocytes were tumor free. In the second, a separate set of mice were injected with LLC1 cells and then modified or unmodified lymphocytes were injected via the tail vein. Of the mice that received modified CD8⁺ lymphocytes, 62.5% remained tumor free whereas, 0% of those receiving unmodified lymphocytes remained tumor free. These activities were also tested in regards to B16-F1 tumour cells. In these experiments, lymphocytes isolated from mice that bore the B16-F1 cancer (the growth of which is also dependent on TGF-β) were divided into one batch which was modified to express AHSG and one batch which was not modified. These lymphocytes were then tested against the LLC1 cancer. The addition of these lymphocytes, arising from mice that were not exposed to LLC1 cells, resulted in neither batch administered to mice with LLC1 cancer having any effect on LLC I cancer growth and none of the mice remained tumor free in either group. These results show that AHSG can block the actions of TGF-β when expressed in a cell which is exposed to the TGF-β growth factor.

Also described are methods for modification of the immune system for purposes of administering an effective cancer cell therapy.

Preferred embodiments employ CDS enriched lymphocyte populations which are transformed with the AHSG gene.

In addition to the ability of AHSG to bind the TGF-β receptor (Demetriou, M. et al J Biol Chem 271:12755 (1996)). AHSG can also inhibit the proteolytic activity of the enzyme, cathepsin B. Cathepsin B is also an activator of the latent form of TGF-β. As TGF-β exists in a larger latent or dormant form but conversion to an active and smaller form requires proteolytic cleavage and many cancer cells are known to express pericellular cathepsin B; cancer cells are able to convert latent TGF-β to its active form, thus enabling the cells to hide from the immune system.

In normal lymphocytes, there is no detectable cathepsin B activity which is consistent with past observations; as cathespin B pressure would result in conversion of present, it would convert latent TGF-β to its active form and cause auto inhibition of the lymphocyte activity. Yet in cancer cells, which utilize TGF-β to evade immune surveillance, the pericellular cathepsin B allows these cells to surround itself with active TGF-β. The recent finding that AHSG has cathepsin B activity is important in defining the mechanisms by which AHSG blocks the actions of TGF-β. This has been demonstrated using lymphocytes altered to express AHSG, which is likely secreted from the cells; and once outside the cell, acts in an autocrine fashion to block the actions of TGF-β. Though an exact understanding of the underlying mechanism is not necessary to practice the present invention, the ability of AHSG to block TGF-β action appears to arise from the combined actions of AHSG to inhibit the activity of cathepsin B and to competitively bind the TGF-β receptor and thus prevent TGF-β from binding to its receptor. In brief, AHSG expressed in lymphocytes has the ability to prevent the activation of TGF-β produced by cancer cells. Lymphocytes have the innate ability to locate cancer cells, but when they arrive at the site, the presence of TGF-β, secreted by the cancer cells, blocks the actions of the wild-type lymphocytes. However, lymphocytes engineered by the approach of the present invention to produce AHSG once they migrate into the microenvironment of tumors that are immunologically anergic arising from the actions of TGF-β, overcome the cancer's ability to escape from the immunological actions of such lymphocytes.

Other proteins belonging to the cystatin family contain such domains and are known to inhibit cystein proteases. Cathepsin B is a member the cystein protease family. However, many proteins containing horology to the cystatin domain have not been shown to have cathepsin B inhibitory activity. AHSG has tandem repeats of a cystatin motif and we discovered that AHSG is an inhibitor of cathepsin B. The ability of AHSG to inhibit the function of cathepsin B has important implications for the regulation of cell growth because cathepsin B on the cell surface is known to activate latent form of TGF-β (Fu, S.Q. et al Blood 89:1460 (1997); Wroblewski, J. M. et al Blood 89:4664 (1997)).

Since it is difficult for adenovirus to insert a transgene into lymphocytes (Fu, S.Q. et al Blood 39:1460 (1997); Wroblewski, J. M. et al Blood 59:4664 (1997)), one embodiment of the present invention provides for improved methods for transforming lymphocytes using the AAV virus. Additional attractive features of this gene delivery system come from its ability to infect a wide range of host cells while inducing a low host immune response, presenting low toxicity coupled with persistent expression which thereby permits production of high levels of the desired protein.

Also described are lymphocytes armed with the capability to express AHSG have the ability to migrate from the site of injection to distally administered and localized LLC1 cells. This approach is completely different from that designed to block TGF-β signaling using gene targeting of the TGF receptor Type-II (Gorelik, L. et al Nature Rev Immunol 2:46(2002)). Lymphocytes have the natural ability to seek out and migrate to sites containing cancerous cells. AHSG expressing lymphocytes in accordance with the present invention have the ability to overcome the cloaking mechanisms used by the cancer cells to escape from the immune system.

A further embodiment of the present invention as defined in the claims provides lymphocytes for the therapeutic administration to a patient of an agent inhibiting the activity of cathepsin B and also blocking the binding of TGF-β with its receptor. The agent are lymphocytes transformed with a nucleic acid which encodes a protein which accomplishes the desired effects, the protein being AHSG. The nucleic acid may be DNA or RNA and may be presented in a carrier like a liposome or a viral vector which itself may have associated further operative elements such as enhancers, promoters and the like.

A further embodiment of the present invention as defined in the claims and relating therapeutic treatment of cancer provides treated lymphocytes of a patient so that they are able to express agents which can inhibit the activity of cathepsin B and also interfere with the binding of TGF-β to its receptor, the agent being AHSG. Such treatment may be accomplished by in vivo gene therapy approaches or ex vivo by transfection of the patient's isolated lymphocytes with nucleic acid encoding for the desired agent and then administering such transformed lymphocytes to the patient.

The accompanying drawings, which are incorporated in and constitute a part of this specification, illustrate preferred embodiments of the present invention and, together with the description, serve to explain the principles of the present invention.

### Brief Description of the Drawings

Further understanding of the various principles and aspects of the present invention may be had by reference to the figures wherein:

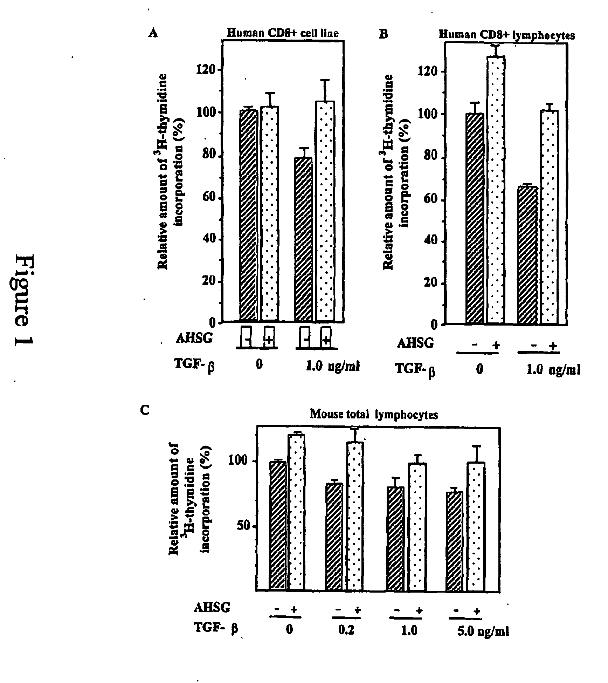

Fig. 1 shows the antagonistic effect of human AHSG on ³[H]-thymidine uptake by lymphocytes or lymphocyte derived cell lines. The effect of TGF-β on cell proliferation as reflected by DNA synthesis was measured using cellular ³[H]-thymidine uptake. The pSRα-AHSG plasmid containing the human AHSG cDNA driven by the SR-α promoter was introduced into the cells (panel A, B and C: T8-IIM a human CD8⁺ cell line, human CD8⁺ lymphocytes from peripheral blood and total mouse lymphocytes, respectively) of interest using a standard transfection method as described later. The relative amount of ³[H]-thymidine uptake incorporated into the DNA was detected and then expressed relative to a fixed amount of protein. Cells that were transfected with the vector carrying AHSG or empty vector were exposed to control media or that containing TGF-β;

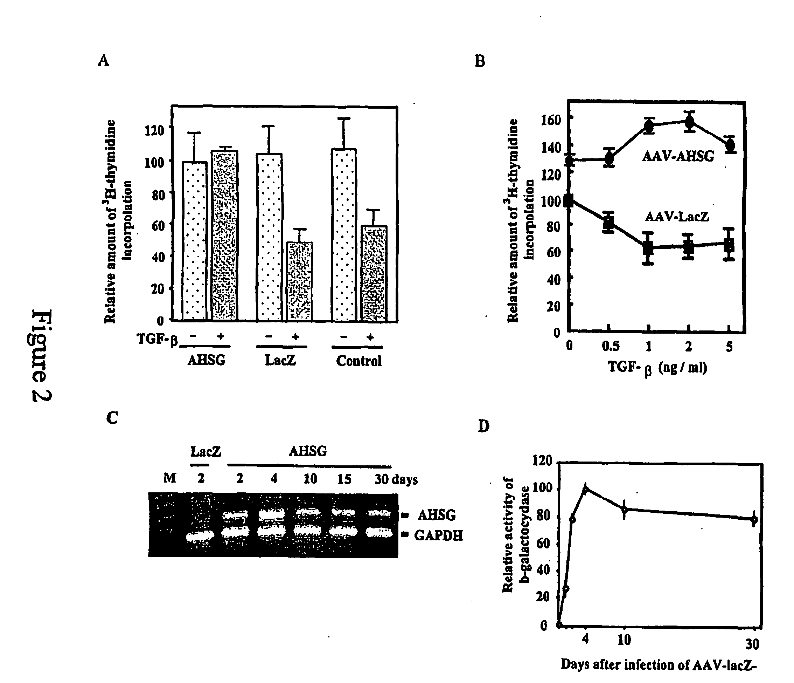

Fig. 2 shows the effect of gene transfer of AHSG using AAV in different cells where Panel A shows the ability of AHSG to block the growth suppressing effects of TGF-β in a human T cell line, Jurkat. Cells were infected with AAV-lacZ (hatched bars) or AAV-AHSG (shaded bars) and then treated with 2 ng/ml of TGF-β. Panel B shows similar studies mouse total lymphocytes. Panel C contains data showing the prolonged expression of AHSG RNA in Jurkat cells that were infected with AAV-AHSG before harvest. The relative amount of AHSG mRNA was detected using RT-PCR and the results showed persistent expression up to 30 days post infection with AAV-AHSG. Expression of GAPDH gene was used as internal control. Panel D shows that expression of Lac Z, monitored using enzymatic activity, behaved in a similar fashion;

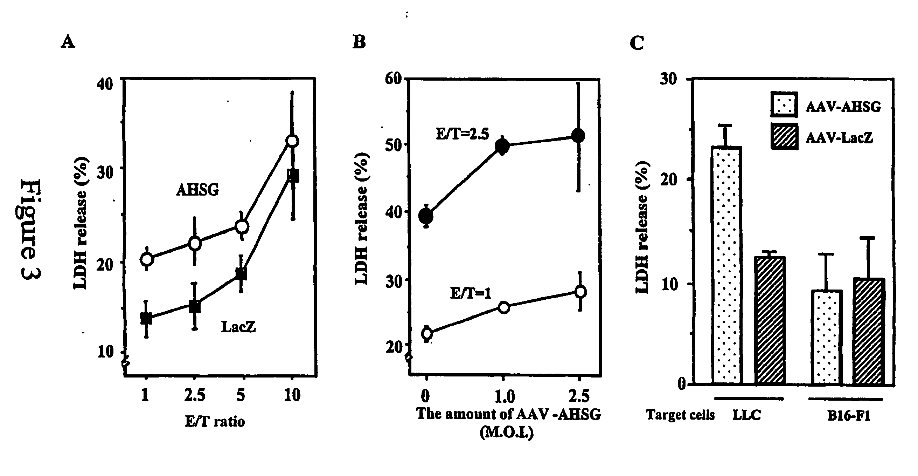

Fig. 3 shows induction of cytotoxic activity by AAV-AHSG using mouse CD8⁺ cells from tumor bearing mice. Panel A shows the cytotoxic activity of lymphocytes infected with AAV-AHSG or AAV-LacZ that target LLC1 cells. The ability of the lymphocytes to lyse the LLC1 cells was measured at different effector 'E' to target 'T' ratios. The M.O.I of AAV used to infect the lymphocytes was 1. The release of LDH from the LLC1 cells served as a marker of cytotoxicity. Panel B shows the ability of increasing the M.O.I. to obtain a more effective lysis at a fixed E/T ratio. Cytotoxic activity was measured versus the amount of virus used for infection of lymphocytes. Increased amounts of virus lead to more lysis at the same E/T ratio. Panel C shows the specificity of the enhanced cytotoxic activity against the LLC1, but not B16-F10 cells. Lymphocytes were isolated from the mouse injected with LLC1 in this experiment. The M.O.I was 1 and the E/T ratio 2.5;

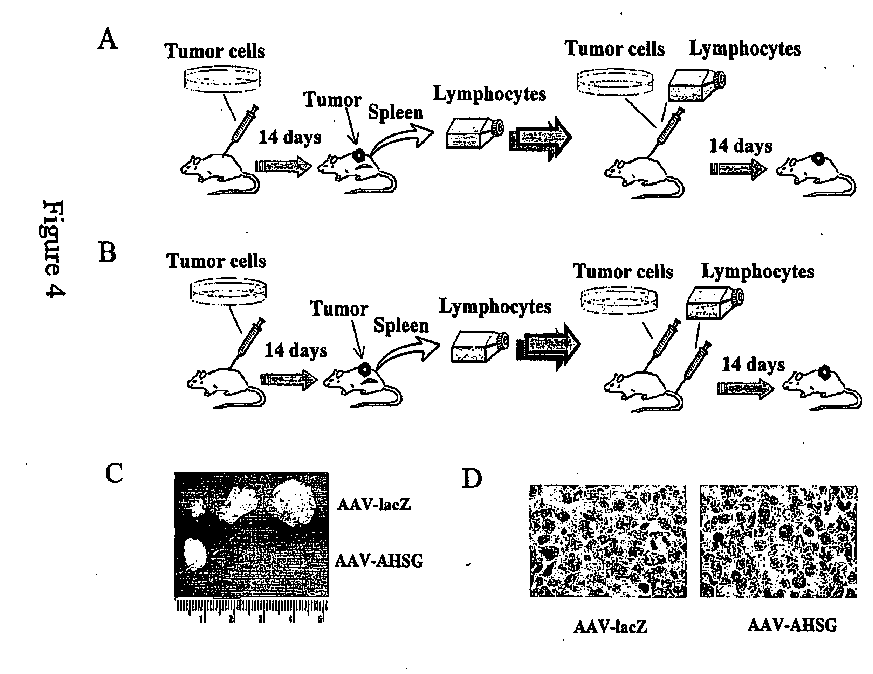

Fig. 4 shows cytotoxic activity in an *in vivo* model Panel A reflects the mixture of LLC1 and lymphocytes from LLC1 bearing mouse and injection at the intrascapular region of mice. Panel B reflects injection of LLC1 cells at the intrascapular region of mice and then the lymphocytes from LLC1 bearing mouse were isolated. These cells were infected with AAV-AHSG or-LacZ prior to injection at the tail vein. Panel C shows the tumor from the injection of animals using the scheme in panel A. Note the lack of tumor in 2 of the mice. Panel D shows the histological features of the tumor from a mouse injected with LLC1 and lymphocytes expressing AAV-LacZ (left panel) or AAV-AHSG (right panel);

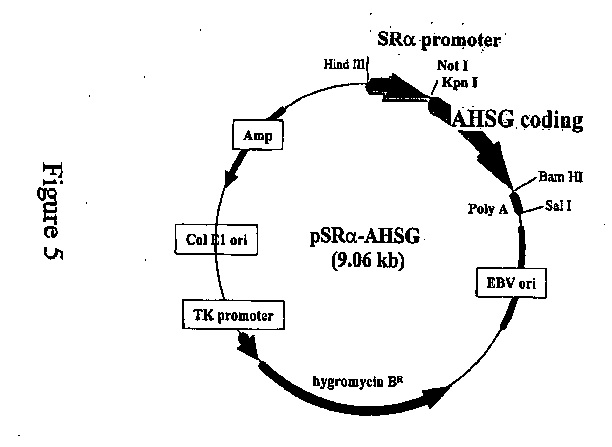

Fig. 5 shows a schematic map of the pSRα-AHSG plasmid;
Fig. 6 shows a schematic map of the pAAV-AHSG plasmid;
Fig. 7 shows relative cathepsin B activity on the cell surface of LLC cells transfected with AHSG expression vectors;
Fig. 8 shows the effect of partially purified AGST on the activity of pericellular cathepsin B;
Fig. 9 shows a gel of RT-PCR products demonstrating the mRNA expression of AHSG and GAPDH from clones A1, A2, C1 and C2;
Fig. 10 shows a graph demonstrating the time-dependence of tumor growth in C57BL mice subcutaneously injected with LLC cells containing either pcDNA3 (Control) or pcDNA-AHSG (AHSG-LLC); and
Fig. 11 shows a graph demonstrating the size of the tumors at day 20 in C57BL mice subcutaneously injected with LLC cells containing either pcDNA3 (Control) or pcDNA-AHSG (AHSG-LLC).

### Detailed Description of the Invention and Best Mode

In accordance with the desired goals of the present invention, our first step was to identify an inhibitor of TGF-β action. We chose to use human AHSG for this purpose because of its potential ability to block the actions of TGF-β through competition for binding to TGF-β receptors.

Prior to using human AHSG to develop a therapy to modify cells of the immune system, we had to be certain of its abilities to block the actions of TGF-β. To confirm this activity, we expressed human AHSG in cells that do not normally express the gene. The first step was to use RT-PCR to amplify from total HepG2 RNA the AHSG sequence and clone this cDNA into an expression vector driven by the SRa promoter. The resulting expression vector was called pSRα-AHSG (see map 1). The pSRα-AHSG construction was then inserted into hematopoietic cells that do not normally express the AHSG protein. The first set of experiments used T8-IIM cells because the growth of these cells is known to be inhibited by TGF-β (Miyamoto, K. et al Jpn J Concer Res 82:1178 (1991)). Results (Fig. 1A) showed that growth of the wild-type cells, as determined by measuring ³[H]-thymidine uptake, was inhibited by exposure to TGF-β. In contrast, the cells transfected with pSRα-AHSG were resistant to the suppressive effects of the TGF-β. These findings showed that cells given the ability to express human AHSG were protected against the growth suppressive action of TGF-β.

The present invention provides, for use in treating human cancers, human lymphocytes made to express AHSG at therapeutically effective levels. In an especially advantageous embodiment, the lymphocytes were enriched for CDS⁺ cells. Such cells may be advantageously isolated from human peripheral lymphocytes using magnetic beads and then transformed with pSRα-AHSG. Like the T8-IIM cells, the CDS⁺ lymphocytes transfected with the human AHSG cDNA were protected against the growth suppressing effects of TGF-β (Fig. 1B). In contrast, lymphocytes carrying the control plasmid behaved exactly like non-transfected wild type cells and when exposed to TGF-β, showed attenuated uptake of ³[H]-thymidine. As a result of the present invention, the function of AHSG can be transferred to human lymphocytes and that the actions of AHSG can block the inhibitory actions of TGF-β on cell growth. The effectiveness of the method in an animal model was confirmed (Fig. 1C).

### Cloning of human AHSG.

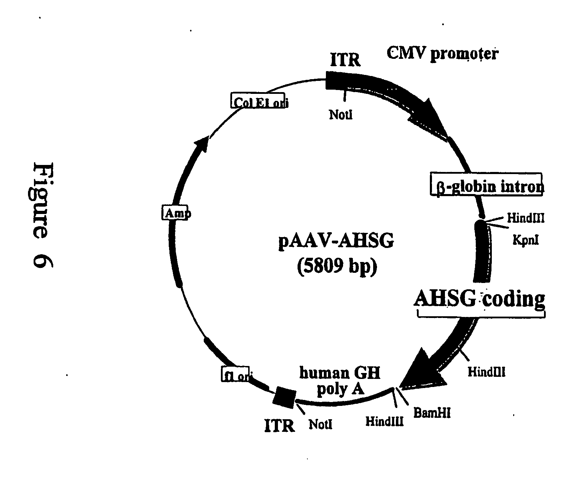

In order to obtain the cDNA encoding human AHSG, RNA was isolated from a cell type (Hep G2) that expressed the gene. Either human liver tissue or cultured cell lines are suitable for the extraction of total RNA. Human fetal bone mallow cells are also reported to express AHSG (Dziegielewska, K. et al Histochem Cell Biol 106:319 (1996)). The isolated total RNA was used in a reverse transcriptase (RT) reaction and then amplified using polymerase chain reaction, PCR (conveniently referred to as RT-PCR hereinafter). The AHSG cDNA was prepared using total RNA extracted from-the hepatoma cell line, HepG2, and the resultant total RNA was used as template in RT-PCR. RNA-from the cells was extracted using the acidic guanidine thiocyanate method. The primer sequences used in the RT-PCR were 5'-ggggtacccatgaagtccctcgtcctgctc-3' and 5'-cgggatccttctgtgccaaacctcctcatc-3'. The 5' ends of these primers contained added sequences that were recognized by the restriction enzymes Kpn I and Bam HI. The addition of these restriction sites enabled insertion of the cDNA product into the cloning vector, pCDL81 to yield pSR-AHSG (see Fig. 6). This vector allowed expression of the AHSG protein driven by the SRα promoter (Takebe, Y. et al Mol Cell Biol 8:466 (1988)) when the plasmid was inserted into cells. The coding region of AHSG coding was confirmed by nucleotide sequence analysis and found to be identical to the reported sequence (Swiss-Prot: P02765).

### Construction and production of AAV-AHSG.

AAV targets cell types that express the receptor for this virus. This receptor is comprised of a complex of heparin sulphate and the fibroblast growth factor, FGF receptor (Qing, K. et al Nat Med 5:71 (1999)). Expression of the AAV receptor is low in CD34⁺ hematopoietic progenitor cells, a feature which many help explain why the efficiency of AAV infection of mouse lymphocytes was less than one tenth the infection rate in Hela cells (data not shown). AAV mediated gene transfer has the potential to increase the infection efficiency against certain types of target cells by modifying its capsid gene (Shi, W. et al Hum Gene Ther 12:1697-(2001)). An added feature of using AAV is that higher titers of the virus particles may be necessary to observe a more robust effect in hematopoietic cells. These features of AAV make it a useful vector for introducing AHSG into target cells.

Prior to testing for AHSG expression, the efficiency of infection was determined using a comparison of two cell types (a non-lymphocyte vs. a lymphocytic cell) transfected with the LacZ gene followed by measurement of β-galactosidase activity using *in situ* staining and plaque forming activity (data not shown). Despite the lower efficiency of gene transfer into lymphocytes using AAV, our data (Fig. 2) showed significant expression of AAV-AHSG. In Jurkat cells, a cell line derived from human T lymphocytes, infection with AAV-AHSG protected it against the suppressive effects of TGF-β (Fig. 2A and 2B), but infection with AAV-LacZ did not. The expression of AHSG mRNA and β-galactosidase activity persisted for at least 30 days post infection with either AAV-AHSG or -LacZ, respectively (Figs. 2C and 2D). These results support the choice of-the AAV for inducing AHSG in the desired target cell for the development of cell therapy for cancer.

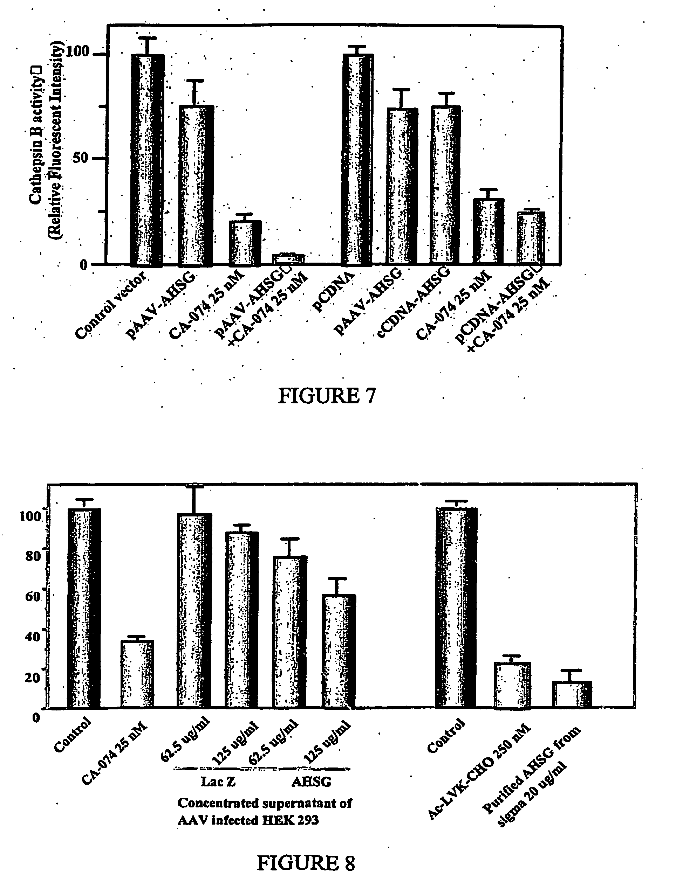

The use of the AAV vectors is described by U.S. Patent Numbers 5,622,856; 5,945,335; 6,001,650; 6,004,797 and 6,027,931, the disclosures of which are fully incorporated herein by reference. The Kpn I and Bam HI fragment was excised from pSR-AHSG and this fragment contained the coding region for AHSG. This fragment was subcloned in pCMV-MCS to yield pAAV-AHSG (see Fig. 7). All plasmid DNAs containing the required elements to produce AAV particles were extracted and purified using a standard alkaline SDS method (Sambrook, J. et al Molecular Cloning: A Laboratory Manual (Second Edition). Cold Spring Harbor Laboratory Press (1989)) and then purified using cesium chloride ultracentrifugation. The plasmid DNAs purified using this method; pAAV-AHSG or pAAV-lacZ, pHelper and pRC were mixed together and co-transfected into HEK293 (from ATCC) cells to produce the desired viral particles. The method used to transfect the DNA was conventional calcium phosphate precipitation. In brief, HEK293 cells were cultured in Dulbecco's modified Eagle's medium, DMEM supplemented with 5% FCS for 3 to 5 days. These cells were collected by scraping cells from the culture vessel and then re-suspended in 1 ml of PBS-MK (PBS to which was added 1mM MgCl₂ and ImM KCI) per dish.

To release AAV particles from cells, cell membranes were disrupted using two cycles of freezing and thawing at -80 *C and 20 °C. The cellular RNA and lysed DNA were digested using a combination of RNase A and DNAse I at a final concentration of 50 ug/ml and 10 µg/ml, respectively. Cellular debris was removed by centrifugation at 1500 g at 4°C. The clarified supernatant was filtered twice. The first filtration used a 5-µm pore size filter (Millipore, SLSV R25 LS Bedford, MA) followed by a 0.8-µm pore size filter (Millipore, SLAA 025 LS). Next, the AAV particles in the filtrate were concentrated using a centrifugation filter device (Millipore Biomax-100K NMWL, UFV2BHK40). The AAV particles were concentrated and retained in the upper portion of the vessel. This material was washed once by adding PBS-MK, pH 7.4, to dilute the AAV concentrate and then spun down again to remove low molecular weight components. The quality of viral stock was sufficient for use to infect cultured cells including lymphocytes. Additional purification may be effected using ultracentrifugation through preformed gradients of iodixanol followed by HPLC on a heparin-agarose column. Performing this added step allowed us to purify the AAV more than 20-fold. The resulting viral preparation was aliquoted and frozen at -80 °C for use in the following experiments. To measure the titer of the virus, the AAV-LacZ was used to infect Hela cells and the abundance of LacZ expression indicated. A typical preparation varied from 2x 10⁷ to 5x 10⁷ plaque forming units (PFU) per ml of AAV. The preparation and isolation of both AAV-AHSG and -lacZ were done in parallel. Since there was no internal reporter gene to measure the titer of AAV-AHSG, it was assumed that the titer of AAV-AHSG and AAV-lacZ were the same due to parallel preparation.

### Isolation of human lymphocyte cells from peripheral blood.

Human peripheral blood mononuclear cells (PBMC) containing T lymphocytes were obtained from healthy adults under informed consent. The desired fraction of the cells enriched for T-lymphocytes were isolated using standard Ficoll-Hypaque gradient centrifugation. The specific population of the lymphocytes may be advantageously separated from PBMC using ferric beads coated with an antibody that is specific to a surface marker on the desired lymphocytes. Cytotoxic T-cells, one of the most important cells for tumor immunity, express a surface marker protein called CD8. CD8⁺ cells were enriched from the total PBMC using Dynabeads coated with an antibody directed against human CD8 (Dynal Inc, NY). Lymphocytes that bound to the beads were collected using a magnet and dissociated from the ferric beads using DNase I to digest a spacer DNA molecule that linked the ferric beads to the specific IgG. Following release of the cells, the residual ferric beads were removed using a magnet. This method helped to enrich for CD8⁺ cells so that the population comprised 90 to 95 % of such cells when assayed using FACS analysis. The resulting population of the cells was called CD8⁺ enriched cells. Total lymphocyte cells or CD8⁺ enriched cells were cultured for 3 days in RPMI containing 2 µg/ml PHA-L, 5% heat-inactivated FCS, 200 units/ml IL-2, 5 µg/ml insulin, 10 µg/ml transferrin and 50 µM β-mercaptoethanol. A lectin from *Phaseolus vulga,* PHA-L, a known mitogen of lymphocytes, was added to the media to stimulate growth initially. Thusly activated lymphocytes can be propagated for up to 14 days in the same media without PHA-L.

### Isolation of mouse lymphocytes from spleen.

. In humans, the PBMC provides a source of lymphocytes but in the mouse, the spleen is a more practical source of such cells. The spleen from a C57BL mouse was excised, minced, squeezed using flat top forceps and suspended in 10 ml of RPMI containing 10% FCS. The cell suspension was allowed to sit for 5 minutes at room temperature to allow the large pieces of the spleen to separate from the suspension of single cells. The supernatant yielded a suspension of single cell lymphocytes that was carefully transferred to another tube. The single cell suspension of lymphocyte cells was collected by centrifugation at 150xg for 5 min. Erythrocytes were removed by centrifugation using a discontinuous gradient of Ficoll-Hypaque. The lymphocytes were collected by removing the band of interest from the gradient and then washed with RPMI containing 5% heat inactivated FCS. These cells were cultured for 3 to 5 days in RPMI containing 4 µg/ml Concanavalin A, 5% heat inactivated FCS, 200 units/ml IL2, 5 µg/ml insulin, 10 µg/ml transferrin and 50 µM β-mercaptoethanol. Concanavalin A is a mitogen for lymphocytes.

### ³[H]-thymidine incorporation assay.

The active form of TGF-β1 (R&D Systems Inc. or BioShop Canada Inc) was added to 5x10⁵ lymphocytes per well in 24 well plates and allowed to incubate for 24 hours. The concentration of TGF-β1 was in the range of from about 0.5 to 10 ng/ml as specified in the figure descriptions. Next, 1 µCi of [3H]-thymidine was added to each 1 ml culture of culture media and the cells were incubated for 4 hours. The radio labeled cells were washed three times with 0.4 ml of PBS and then suspended in the same volume of 10% trichloroacetic acid. The TCA fixed cells were washed three times with 0.4 ml of 5 % trichloroacetic acid and two times with 0.4 ml of 70% ethanol. The precipitated cells were dissolved in 0.1 ml of 0.1 N sodium hydroxide and neutralized using the same amount of 0.1 N hydrochloric acid. The amount of 3H-thymidine incorporated in the cells was counted by liquid scintillation counter.

### Detection of AHSG mRNA expression:

To detect whether AHSG was expressed, the AAV-AHSG infected cells were harvested at 3 days following exposure to the virus. Total cellular RNA was isolated from the cells using the method described above. RT-PCR with total RNA from infected cells was used to assess expression of AHSG. Finding AHSG cDNA in the reaction indicated the presence of expression. The primer sequences used in this reaction were 5'-tccaaacacagcccgtgacctc -3' and 5'- ctcatctctgccatgtctagcc-3'. This set of primers is specific for human AHSG mRNA. The GAPDH was targeted as the internal control and the primers used to detect the human GAPDH mRNA were 5'-caccaactgcttagcacccc-3' and 5'-tgaagtcagaggagaccäcc-3'.

### Cytotoxic assays.

Lewis lung carcinoma (LLC1) is the most cancerous cell type in mice. LLC1 cells are tumorgenic in C57BL mice. These cells can grow tumors of more than 5 mm in diameter when 5x10⁴ of LLC1 cells are injected into the intrascapular region of mice. At 10 to 14 days following the injection, LLC1 tumor can be detected by inspection of the injected area. This tumor model has been previously described (Budzynski, W. Arch Immunol Ther Exp (Warsz) 30:363 (1982)).

In order to determine the ability of the lymphocytes infected with AAV-LacZ or -AHSG to affect LLC1 tumor growth, we mixed either type of lymphocyte with 5x10⁴ LLC1 cells and then injected the mixture into the intrascapular region of mice. In these experiments we used either total or CD8⁺ enriched lymphocytes isolated from the spleen of tumor (LLCI)-bearing mice. These cells were cultured as previously described. The lymphocytes infected with AAV-AHSG or AAV-lacZ were cultured for 3 days to allow the virus to express either LacZ or AHSG. At the end of the 3 days, the infected cells were isolated and then mixed with 5x 10⁴ LLC1 cells before injection.

The aim of these studies was to test the cytotoxic capacity of the lymphocytes to lyse the target cells. We refer to the cytotoxic cells as the "effector" and the tumor cells as the "target". Both *in vitro* and *in vivo* studies were performed. For the *in vitro* studies, the cytotoxic activity of lymphocytes was measured by following lactate dehydrogenase, LDH, release from the cells exposed to the effector.

In these studies, lymphocytes were mixed with LLC1 cells and upon lysis of the carcinoma cells *in vitro, .* LDH was released and subject to measurement using a standard colorimetric assay. The target LLC I cells were maintained in DMEM with the supplement of 5% FCS. Target LLC1 cells were seeded in v-shaped 96 well plates at a cell number of 1 x 10⁴ in a volume of 100 µl. The lymphocytes were added to each well and centrifuged at 1,000xg for 3 minutes to insure that the effector cells contacted the target cells. The effector to target ratio, or E/T ratio, for most studies ranged from 1 to 200 but a lower range from 1 to 10 was preferred. To assess the interaction of effector on target cells, the cells were incubated for 4 hours at 37° C in 5 % CO₂ incubator and LDH activity in the media measured using the CytoTox96® kit (Promega corporation) according to manufacturer's protocol.

In addition to the *in vitro* assays, it was also desirable to test the cytotoxic activity of the lymphocytes *in vivo.* For these *in vivo* studies, we injected the effector and target cells into C57BL mice. 5x10⁵/ml of lymphocytes were infected with 1x10⁶ PFU of AAV-AHSG or AAV lacZ. The infected cells were cultured for 3 days followed by the addition of fresh media containing 200 u/ml of IL2 and 2 ug/ml of ConA. 2x10⁵ of lymphocytes were mixed together with 2x10⁴ of LLC1 cells before subcutaneous injection in the intrascapular region. The growth of tumor was monitored for at least 14 days and then the mice sacrificed on the last day. The tumor size was measured using a caliper and the diameter recorded. The mice that did not grow tumors were monitored for up to 2 additional months after the injection of the LLC1 cells to confirm non-growth.

### TGF-β antagonistic effect of AHSG expression in human hematopoietic cells.

Bovine fetuin is predicted to have TGF-β antagonistic activity. To examine whether this possibility is associated with human AHSG we utilized SRα promoter to express the cDNA that encodes the AHSG protein. SRα is comprised of the simian virus 40 early promoter and the R-U5 segment of human T-cell leukemia virus type 1 long terminal repeat (Takebe, Y. et al Mol Cell Biol 8:466 (1988)). We chose this promoter because when it was used to drive the expression of. IL-2, there was 10 times more IL-2 expression compared to another frequently used sequence, the CMV promoter (Tsang, T.C. et al Int J Mol Med 5:295 (2000)). A clone was created comprised of the human AHSG cDNA fused to the SRα promoter to yield pSRα-AHSG (see Fig. 6).

We inserted the cDNA encoding human AHSG into a hematopoietic cell line, which does not normally express the gene. In order to be useful as cell based cancer therapy, cellular expression of AHSG must have the ability to antagonize the effects of TGF-β. For these experiments, we chose the T8-IIM cells because this is a CD8⁺ cell line and its immune function is dependent on TGF-β. Exposure of unmodified T8-IIM cells to TGF-β suppressed their ability to incorporate ³[H]-thymidine to 79±4% when compared to control. In contrast, exposure of the T8-IIM cells transfected with pSR-AHSG completely abrogated the growth suppressive effects of TGF-β (Fig. 1A). In these cells, the incorporation of ³[H]-thymidine was the same as that from cells not exposed to TGF-β. This finding meant that the expression of AHSG blocked the suppressing effects of TGF-β. Since TGF-β expressing cancer cells use this mechanism to escape detection from immune system cells, this observation supported our goal to alter the inherent activity of CTLs to thus by-pass the cloaking mechanisms used by cancer cells.

Next we tested the expression of pSRα-AHSG in CD8⁺ cells enriched from human peripheral lymphocytes. In these experiments, we used magnetic beads coated with an antibody against CD8⁺ to enrich CD8⁺ cells from the peripheral blood. The exposure of native CD8⁺ cells to TGF-β lowered their ability to incorporate ³[H]-thymidine to 63 +/- 2 % compared to control treated cells. In contrast, when pSR-AHSG was transfected into the CD8⁺ cells it augmented the ability of these cells to take up ³[H]-thymidine (Fig. 1B) that was equal to control cells not expressing AHSG or exposed to TGF-β.

Although we expected that expression of AHSG would not affect ³[H]-thymidine incorporation, we speculate that this observation may be due to endogenous secretion of TGF-β from the CD8⁺ T cells. Thus AHSG expression leading to suppression of endogenous TGF-β expression could account for the observation. The addition of TGF-β suppressed their ability to incorporate ³[H]-thymidine and over expression of AHSG antagonized this effect of the growth factor. However, ³[H]-thymidine incorporation did not recover to the level of that of cells transfected with AHSG without treatment of TGF-β. There are two potential explanations for these findings. One, exogenous TGF-β may trigger other suppressive cytokines such as IL-10 (Fiorentino, D.F. et al J Immunol 147:3815 (1991); Taga, K. et al Blood 81:2964 (1993)). The actions of AHSG are specific and appear limited to interfere with the actions of TGF-β but do not affect the actions of other cytokines. Two, another possible explanation is that TGF-β induced apoptosis of the cells and the fragmented cells were not removed during the ³[H]-thymidine incorporation assay. However, regardless of the mechanism, it is apparent that AHSG permits the growth of lymphocytes in the presence of TGF-β.

### AHSG expression in mouse spleen cells antagonizes effects of TGF-β

Next, we wanted to extend the observations arising from the use of the human cell line and primary cultured human lymphocytes to an animal model. In preparation for these studies, we chose to use mice as a convenient animal model for testing whether AHSG suppression of TGF-β signal may be useful for cancer therapy. The purpose of the following studies was to examine whether the activity of human AHSG in immune cells from an animal behave like that of human cells. Therefore, we expressed the human AHSG cDNA in a mouse derived lymphoid cell to examine its effectiveness as an antagonist of the TGF-β effect.

In these studies, mouse spleen cells were treated with IL-2 and concanavalin A to stimulate the proliferation of T-cell lymphocytes. These activated. T-cell lymphocytes were utilized to examine the effect of AHSG. As shown in Fig. 1C, the exposure of control cells to TGF-β lowered their ability to incorporate ³[H]-thymidine to 79% (compare columns 1 and 3) compared to untreated cells. However, the addition of higher concentrations of TGF-β did not suppress further their ability to take up thymidine (Fig. 1C, odd columns). In mouse T-cell lymphocytes transfected with pSRα-AHSG, the expression of the gene increased ³[H]-thymidine incorporation to 118% compared to the non-transfected controls (Fig. 1C, compare columns 1 and 2). Additionally, the transfected cells that expressed AHSG did not have the expected effect of the actions of TGF-β. The exposure of these cells to progressively higher doses of TGF-β failed to bring ³[H]-thymidine uptake to below 100% compared to control cells (Fig. 1C. compare even with odd lanes). Note that in the AHSG expressing cells, the uptake of thymidine was always higher than that in the control cells treated with the identical dose of TGF-β. Together these results show that mouse spleen T cell lymphocytes behave much like that of the human CD8⁺ cell line and peripheral CTLs in their response to TGF-β. Equally important is the observation that equipping these cells with the ability to express AHSG also blocked their response to TGF-β.

### AAV mediated gene transfer of AHSG.

Although the previous experiments provided useful results in both human and mouse cells using transient transfection of AHSG cDNA, one may employ more efficient mechanisms to express a foreign protein in a host cell. For example, there is recent interest in the use of adeno associated virus (AAV) for gene delivery. The results of our recent study show that the innate immune response is transient and minimal compared to that of adenovirus vectors (Zaiss, A.K. et al J Virol 76:4580.(2002)). Another well known feature of AAV is it ability to infect a wide spectrum of hosts and cell types. In addition, AAV vectors have a high efficiency in transducing lymphoid cells (Zhou, S.Z. et al J Exp Med 179:1867 (1994)). Equally attractive is that expression of genes using AAV persists for a long time because of its potential ability to incorporate into the host genome. (Lebowski, J.S. et al Mol Cell Biol 8:3988 (1988)).

Human AHSG cDNA was cloned into an AAV vector to yield pAAV-AHSG (see Fig. 7). The virus particles created from this construct were used to infect Jurkat cells. Jurkat cells are a human T-lymphocyte line derived from an acute T-cell leukemia and serves as a convenient host cell to test for the ability of AHSG to suppress TGF-β activity but also to assess the duration of expression of the gene following infection with AAV-AHSG.

Results (see Fig. 2A) showed that exposure of control cells to TGF-β lowered to 57% their ability to incorporate ³[H]-thymidine (compare columns 5 and 6). Similarly, the Jurkat cells infected with AAV that carried the β-galactosidase gene, *lacZ,* behaved like the control cells. The actions of LacZ should not affect function of the Jurkat cells or the actions of TGF-β. When AAV-LacZ infected Jurkat cells were exposed to TGF-β, ³[H]-thymidine uptake 46% (Fig. 2A, compare columns 3 and 4) was blocked when compared to the uptake by AAV-LacZ infected cells which were not exposed to TGF-β. Like the other cells transfected with the AHSG cDNA, the Jurkat cells infected with AAV-AHSG were unresponsive to the actions of TGF-β. Exposure of the AAV-AHSG infected Jurkat cells to TGF-β failed to affect uptake of ³[H]-thymidine (Fig. 2A, compare column 1 and 2). Together these results show that the Jurkat cells behave much like the human CD8⁺ cell line, peripheral CTLs and mouse total lymphocytes in their response to TGF-β. We unexpectedly observed that equipping Jurkat, or any one of the cells we tested, with the ability to express AHSG blocked the response of such cells to TGF-β.

The studies in the following experiment were designed to determine whether the actions of TGF-β on Jurkat cells were dose dependent. Additionally, we also examined whether the use of AAV to express AHSG afforded these cells protection from increasing doses of TGF-β. First, we infected one set of Jurkat cells with AAV-lacZ and these served as the control (Fig. 2B). When the lacZ expressing cells were exposed to progressively higher doses of TGF-β from 0 to 5 ng/ml of culture media, where was a dose dependent suppression of ³[H]-thymidine uptake that reached a nadir equal to 60% of that in control cells (dose of 1 ng/ml of TGF-β).

Next, the experiment was repeated in mouse spleen T-cell lymphocytes infected with AAV-AHSG. The expression of AHSG in these cells was associated with an increase in the ³[H]-thymidine uptake that reached 125% above the control lacZ expressing cells. Additionally, in the AHSG expressing cells, the addition of TGF-β not only failed to suppress the ³[H]-thymidine uptake, but there was a small dose dependent increase in 3[H]-thymidine uptake to a maximum of 155% above the control lacZ expressing cells at a TGF-β dose of 1 ng/ml. The augmentation of thymidine uptake in the AHSG expressing cells was similar to that observed in the mouse total lymphocytes described above.

### Persistence ofAA V mediated gene transfer.

To determine the persistence of expression of AHSG over time, we measured, using RT-PCR, the presence of AHSG mRNA in Jurkat cells infected with either AAV carrying the lacZ or AHSG gene. Results (Fig. 2C and D) showed that expression of the AHSG expression persisted up to the final test time of 30 days after infection. The relative degree of expression was highest at 4 days after infection and then appeared to plateau at about 15 days. Expression at 30 days ' was not much different from that at 15 days. These findings show that use of AAV is an efficient way of enabling cells to express AHSG and that expression seems to persist for at least 30 days after infection. More importantly the expression of AHSG in these cells gave them the ability to overcome the growth suppressing effects of TGF-β. Thus, we have unexpectedly discovered that human AHSG antagonizes the effects of TGF-β when expressed in Jurkat cells.

### Activation of cytotoxic activity using AAV-AHSG.

The preceding data shows that AHSG has the ability to block the actions of TGF-β and thus permits the proliferation of lymphocytes from human and mouse. Whether the expression of AHSG in the T-cells altered their cytotoxicity, an activity that is essential for developing an anti-cancer therapy, was not known. For these studies, we chose the Lewis lung carcinoma (LLC1) cells, which are know to secret TGF-β in direct correlation to the cells' malignancy potential (Perrotti, D. et al Anticancer Res 10:1587 (1990)).

If tumor cells are immunogenic, then lymphocytes from tumor bearing mouse should exhibit cytotoxic activity against the cancerous cells. However, because the tumor cells were tumorigenic, they possessed a mechanism(s) to escape from immune surveillance. For explanatory purposes, in the following experiments, we labeled the lymphocytes "effector (E)" cells because of their cytotoxic activity and the original tumor cells as the "target (T)" cells. In order to determine the efficiency of the E cells to lyse the T cells, we compared cytotoxic activity using the ratio of E to T cells, i.e. the E/T ratio. The relative cytotoxic activity at a low E/T ratio suggests that the lymphocytes are effective in lysing the T cells. Specificity of lymphocytes was determined by comparing the cytotoxic activity using different Target cells. In the current experiments, lymphocytes were isolated from mice carrying an LLC tumor and accordingly, the lymphocytes or E cells should be specific for the Target or LLC cells. The E cells should have cytotoxic activity against LLC1 but not against other Target cells, if confirmed, then the cytotoxic activity was specific against the Target cells.

In the first series of experiments, we implanted LLC1 cells subcutaneously in the intrascapular region of mice. This procedure exposed the immune system of the injected mice to the LLC1 cells. The LLC1 exposed mice served as a source of lymphocytes when the animals were sacrificed, and the lymphocytes isolated from the spleen of these mice. The cells derived from the spleen acted as the E cells and LLC1 served as the Target for these cells. In order to assess the cytotoxic activity of the lymphocytes, they were treated in the following fashion. Lymphocytes were isolated from the spleen, transformed with AAV-Lac Z or AAV-AHSG and then cultured for 3 days as described below. The cultured lymphocytes were harvested and mixed with LLC1 for 4 hours prior to assay. At the end of the incubation, the amount of LDH released into the media was measured according to the manufacturer's procedure (CytoTox96® Non-Radioactive Cytotoxicity Assay, Promega Corporation).

As expected, when more E cells of either AAV-lacZ or AHSG infected were mixed with LLC1 cells, more LDH was released from the target cells (Fig. 3A). The unexpected discovery was that at any E/T ratio using E cells infected with AAV-AHSG had significantly higher cytotoxic activity, i.e. greater LDH release, compared to control lymphocytes transformed with AAV-lacZ (Fig. 3A, top line is that of the AHSG expressing cells), at an E/T ratio of 1. This meant that when an equal number of lymphocytes and LLC1 were mixed together, an E/T ratio of 1, the AAV-AHSG enhanced killing activity from 14.2+/- 1.8% to 20.6 +/- 0.9 % within 4 hours. Furthermore, the cytotoxic activity appeared to correlate with the amount of AAV-AHSG expressed in the lymphocytes using the E/T ratios tested here (Fig. 3B).

LDH release, compared to control lymphocytes transformed with AAV-lacZ (Fig. 3A, top line is that of the AHSG expressing cells), had an E/T ratio of 1. This meant that when an equal number of lymphocytes and LLC1 were mixed together (an E/T ratio of 1), the AAV-LacZ infected lymphocytes killed 14.2+/- 1.8% of LLC1 in 4 hours. On the other hand, AAV-AHSG infected counterparts killed 20.6 +/- 0.9 % of LLC1. The augmentation of cytotoxic activity was observed at various E/T ratios. Furthermore, the cytotoxic activity appeared to correlate with the amount of AAV-AHSG expressed in the lymphocytes using the E/T ratios tested (Fig. 3B).

Since the T-cells were derived from mice injected with LLC1, they were expected to have specificity for recognizing only tumor cells of this type and not other tumor cells. To confirm this, we measured cytotoxic activity of lymphocytes from LLC1 injected mice exposed to LLC1 or B16-F1 cells, a malignant melanoma cell line from the same strain of mouse (Fig. 3C). The results showed that T-cells derived from the LLC1 injected mouse and given the ability to express lacZ demonstrated a level of cytotoxic activity towards the LLC1 cells similar to that against the B16-F1 cells. The expression of AHSG in these T-cells failed to augment their ability to attack the B16-F1 cells but this modification augmented their cytotoxic activity to target LLC1 cells such that it was two-fold higher than in the control gene, lacZ. These results clearly demonstrated the unexpected discovery that AAV-AHSG has the ability to enhance specific cytotoxic activity of lymphocytes even at relatively low E/T ratios. More importantly, while AHSG expression augments the cytotoxic activity of T cells towards the LLC1 or inoculating cell type, it does not do so against an unrelated cancer cell type.

That equipping lymphocytes cells with the ability to express AHSG enhanced their cytotoxic ability was further demonstrated using the release of LDH from tumor cells. These experiments used lymphocytes isolated from the spleen of mice that were injected with LLC1. The lymphocytes from these mice were treated with mitogens and then infected with AAV-AHSG or AAV-LacZ prior to mixing with LLC1 or a non-related B16-F1 cancer cells. The results (Fig. 3) showed that lymphocytes infected with AAV-AHSG enhanced their cytotoxic activity. The actions of AHSG were evident even at a low E/T ratio. Furthermore, the enhanced cytotoxic activity was specifically targeted towards the original cancerous LLC1 cells, the same cells that were injected into the mice from which the lymphocytes were derived. However, this cytotoxic activity did not extend to the B16-F1 cancer cells. One route by which an inflammation reaction may take place is believed to be a non-specific immune response. The fact that the instant approach specifically enhances the cytotoxicity of the lymphocytes towards the original cancer cells makes it an attractive approach for autologous immune therapy.

### Cytotoxic activity in vivo.

LLC1 cells are reported to be highly tumorgenic, but these cells have weak metastatic potential in mice. We tested the *in vivo* cytotoxic capacity of T-cell lymphocytes transformed with AAV-AHSG against LLC1 cells in steps. The first step was to maximize interaction of the T-cell lymphocytes with the LLC1 cells by mixing the two cell types together before injecting subcutaneously. For these studies 2x 10⁴ of LLC1 cells were mixed with 5x10⁵ T-cells transformed with either AAV-LacZ or with AAV-AHSG and then injected subcutaneously at the interscapular region of mice.

In previous studies, the injection of LLC1 cells produced a tumor at the injection site within 7 to10 days following administration (Budzynski, W. Arch Immunol Ther Exp (Warsz) 30:363 (1982)). Our first goal was to determine whether there was a differential cytotoxic activity of T-cell lymphocytes transformed with AAV-LacZ or AAV-AHSG and whether such activity affected LLC1 tumor growth (Table I). Six mice were injected with LLC1 cells and lymphocytes carrying AAV-lacZ. At the end of 14 days, all mice (6/6 or 100%) had significant tumor growth (3 of these are shown in Fig. 4). In contrast, 4 of the 6 mice (or 67%) that were injected with LLC1 and lymphocytes carrying AAV-AHSG had no tumor growth (1 of the 6 shown in Fig. 4C). Furthermore, in one of the two mice that had tumor growth, the tumor was small (< 5 mm as compared to 15 mm for control mice). The color of the tumor was white and this might have reflected the low abundance of angiogenic tissue surrounding the tumor. The histological analysis of the small tumor suggested a more benign phenotype because of a low mitotic index and a lower density of the chromosome (Fig. 4D). The size of the lesion in the other mouse that grew a tumor, was not different from that of the controls. Together these results demonstrate that AAV-AHSG transformation of T-cell lymphocytes enhances their cytotoxic activity and can decrease the tumorgenic potential of LLC1 cells when *in vivo*. Mixing of the LLC1 and lymphocytes together insured that the two different cell types were in direct contact with each other.

**Table 1, in vivo studies using LLC1 tumors and total or CD8⁺ cells injected into mice.**

| Tumor | Lymphocyte and Tumor cell mode of injection | LacZ | AHSG | Survival rate of AHSG infected lymphocytes injected mouse | Comments |
|---|---|---|---|---|---|
| LLC1 | Total lymphocytes mixed with LLC1 & injected, subcutaneous | 0/8 | 6*¹/10 | 60 % | one mouse has just very small tumor at the day 18 |
| | | | | | |
| | Total lymphocytes injected i.v. and tumor injected SQ, same day | 0/4 | 3*²/12 | 25 % | one mouse needed 10 more days for the tumor to grow to 5 mm size |
| | | | | | |
| | CD8⁺ lymphocytes mixed with LLC1 & injected, subcutaneous | 0/4 | 2/4 | 50 % | |
| | CD8⁺ lymphocytes injected i.v. and tumor injected SQ, same day | 0/6 | 5/8 | 62.5% | |

The results show the number of mice and percentage of mice that did not grow tumor after 10 days.

LLC1; 2x10⁴ cells / mouse, lymphocytes; 5x10⁵ cells / one injection

### Injecting LLC1 cells subcutaneously and lymphocytes IV.

The preceding experiments showed the efficiency of the AAV-AHSG in altering the cytotoxic activity of the lymphocytes and enhancing their ability to destroy LLC1s. The design of this experiment mimicked optimistic conditions because it permitted lymphocytic destruction of the tumor at an early stage. To create a scenario that more realistically reflects a model of tumor development, we injected 2x10⁴ LLC1 cells subcutaneously into mice and then 4 days later injected via the mouse's tail vein, 5x10⁵ T-cell lymphocytes transformed with either AAV-lacZ or AAV-AHSG. In the first set of experiments, total lymphocytes (meaning not just the enriched CD8⁺ lymphocytes) were used for the injection. The results from these studies showed that all 4 animals (100%) injected first with LLC1 and then with lymphocytes transformed with AAV-lacZ grew tumors. Unlike the previous experiment, 8 out of the 12 (75%) mice injected with LLC1 and lymphocytes transformed with AAV-AHSG grew a tumor at 14 days. This finding meant that only 25% of the mice were protected and did not grow a tumor.

We postulate that the low level of protection against tumor growth in this experimental design arose from one of at least two possibilities. One possible explanation is that the cytotoxic cells could not reach the tumor site and the second possible explanation is that the number of cells with enhanced cytotoxic activity was not sufficient to reach therapeutic effectiveness. In order to augment the relative cytotoxic activity of the injected cells to address this situation, we used magnetic beads to isolate CD8⁺ cells from the total lymphocytes and used these isolated cells in the injections. All 6 out of 6 control mice injected with LLC1 and 5x10⁵ CD8⁺ lymphocytes transformed with AAV-lacZ, mice developed tumors. In contrast, 5 (62.5%) of the 8 animals injected with the AAV-AHSG transformed lymphocytes did not develop any tumor. This unexpectedly demonstrated that CD8⁺ enriched lymphocytes appear to augment the cytotoxic activity induced by AAV-AHSG transformation.

### Lymphocytes modified with AHSG cDNA are specific for LLC1 but not B6-F1.

In the current model of cell based therapy for TGF-β dependent LLC1 cells, we have discovered that AHSG expression in T-cell lymphocytes imparts to such cells the specificity to recognize cancer cells that use TGF-β to hide from the immune system. This was confirmed using the same approach as above to inject B16-F1 cancer cells at the intrascapular region of mice followed by i.v. injection on the same day of T-cell lymphocytes, transformed with either AAV-lacZ or AAV-AHSG. Results of these studies showed that all mice injected with B16-F1 and the AAV-lacZ transformed T-cells grew tumors. In addition, all mice injected with B16-F1 and AAV-AHSG transformed T-cell lymphocytes also grew tumors. A further experiment which used the magnetic bead technique to use enrich CD8⁺ lymphocytes which were then transformed with either AAV-lacZ or AAV-AHSG showed no alteration of the rate of tumor growth. All mice injected with either type of T-cell lymphocytes grew tumor. These findings suggest that the cytotoxic activity of T-cell lymphocytes which have been transformed to express AHSG appear limited to cells which are TGF-β dependent like LLC1 cells but not those which are TGF-β independent, such as B16-F1 cancer cells.

### Effects of AHSG on Cathespin B Activity

In this example we used the fluorogenic substrate called z-Arg-Arg-AMC. This peptide does not enter into the cell and is specific for assaying cathepsin B activity. The ability of this substrate to stay outside the cell allowed us to measure the enzymatic activity of pericellular cathepsin B.² Many human and mouse cancer cell lines have cathepsin B activity on the cell surface.

To measure the effect of AHSG on the activity of cathepsin B, we expressed AHSG using expression vectors, pAAV-AHSG or pCDNA-AHSG that were used to transfect Lewis lung carcinoma (LLC) cells. These cells normally express cathepsin B (Hulkower, K. I. et at Eur J Biochem 267:4165 (2000)). Thus the addition of the substrate to the cells transfected with a non-specific vector lead to lysis of the peptide and the resulting level of cathepsin B. activity was arbitrarily set at 100 (Fig. 7, left column). Cathepsin B activity following transfection with pAAV-AHSG was roughly 25% lower than that in control cells (Fig. 7, second column). Next 25 nM of a known inhibitor of cathepsin B, CA 074 was added to the assay. The presence of this agent lead to the expected inhibition of cathepsin B activity (Fig. 7, third column). Not surprisingly, the inhibition of cathepsin B was highest in connection with cells that were transfected with pAAV-AHSG and also exposed to CA-074 (Fig. 7, fourth column). Despite the low transfection efficiency of LLC cells that ranged from 10 to 20% as measured using *in situ* staining of β-galactosidase activity, the observed reduction of the cathepsin B in the presence of AHSG supports the present invention that expression of AHSG suppresses cathepsin B activity on the cell surface.

A separate set of studies (Fig. 7, column 5-9) that used another expression vector pcDNA (purchased from Invitrogen, Carlsbad, CA) to express AHSG showed similar results. The results showed that spent media from cells transfected with the empty vector, pCDNA did not inhibit cathepsin B activity. In contrast, the use of the pAAV-AHSG construct inhibited cathepsin B by roughly 25% (Fig. 7, column 6) and a similar inhibition was observed in media from cells transfected with pcDNA-AHSG (Fig. 7, column 7). As expected the presence of 25 nM CA-074 inhibited cathepsin B activity (Fig. 7, column 8) and the combination of both the pcDNA-AHSG and CA-074 further reduced cathepsin B activity (Fig. 7, column 9). Due to variable background of fluorescence in this experiment, columns 8 to 9 showed less inhibition compared to columns 3
²(Hulkower, K. I. et al Eur J. Biochem 267:4165 (2000)). to 4. The level of the background was slightly different because of differing preparation of the cells. Together these studies show that expression of AHSG in the cells had cathepsin B blocking activity.

To be certain that this observation was not limited to LLC cells, we also assessed the inhibitory activity of AHSG on cathepsin B contained in the supernatant of HEK293 cells that were infected with AAV-AHSG. In this experiment, we collected spent media from HEK293 cells that did and did not express AHSG. The reason for using the AAV virus is because it enabled more efficient expression of AHSG in the HEK293 cells. The HEK293 cells are typically used for the production of virus and should also express the transgene contained in the vector. Results (Fig. 8) showed that in cells infected with pAAV-LacZ, the cathepsin B inhibitory activity was non-existent and set at 100-("Control" columns). As shown in studies above, the presence of CA-074 inhibited activity of cathepsin B. The use of spent media from HEK293 cells infected with AAV-AHSG or -LacZ were assayed for AHSG inhibitory activity. As expected, purchased AHSG (Sigma, St Louis, MO) that was partially purified from human plasma had the predicted TGF-β inhibitory activity (Fig. 8, column 9). The spent media from AAV-AHSG infected HEK293 cells was partially purified and concentrated. The addition of concentrated media containing AHSG from the AAV-AHSG virus infected HEK293 cells (Fig. 2, column 5 & 6) and the AHSG purchased from Sigma showed significant inhibition of pericellular cathepsin B activity (Figure 8, column 9). Furthermore, the doubling of the media concentration yielded a 2-fold increase of TGF-β inhibitory activity. In other words, the presence of 62.5 or 125 ug/ml of the AHSG containing media lead to a 23 and 46% reduction, respectively in cathepsin B activity (Figure 8, column 5 & 6).

A slight inhibition of cathepsin B observed in the negative control cells infected with AAV-lac Z (Fig. 8, column 3 & 4) may have been due to endogenous fetuin contained in the bovine serum that was a component of the culture media. Each lot of purchased AHSG had variable inhibitory activity of pericellular cathepsin B. However one of the lots of AHSG inhibited completely the activity of cathepsin B but the amount of protein required was less than 10% of the concentration of AHSG in the blood of adults (0.3 to 0.6mg/ml). AHSG is modified by glycosylation and phosphorylation. It is possible that these modifications affected the inhibitory activity of cathepsin B and these modifications on the protein are likely variable from one preparation to the next of extracted AHSG.

### Material and Methods

### Preparation of the cells

The optimum condition to detect pericellular cathepsin B is in rapidly growing cells. To achieve this condition, 4x10⁶ cells were seeded in a 60mm dish and used for cathepsin B assay on the next day. Transfection of the LLC cells was performed in suspension condition using Superfect (Qiagen N.V., KJ Venlo, The Netherland) according to manufacturers instructions. The monolayer cells on the 60 mm dish were washed twice with PBS containing 1 mM EDTA and then suspended in 0.8 ml of regular growth media (D-MEM supplemented with 10% FCS). The cells sat for 5 minutes to precipitate the big clumps of cell aggregates and then 0.6ml of the top layer of the cell suspension was transferred to a 5ml polystyrene tube. Superfect-DNA complex was produced by adding 30 µl of Superfect to a 150 µl of D-MEM containing 5 µg of the plasmid DNA. This suspension was allowed to sit for 10 minutes at room temperature and then added to 0.6ml of the single cell suspension. The DNA and cell mixture was incubated for 3 hours in a CO₂ incubator followed by two washes with PBS and then cultured in regular growth media (D-MEM supplemented with 10% FCS) overnight. The next day cells were subcultured and 4x10⁶ cells that then seeded onto a 60mm dish and these transfected cells were grown overnight prior to use in the cathepsin B assay. The cells from a 60mm dish were removed from the dish by vigorous pipetting with 4ml PAB buffer (Hank's balanced salt solution without sodium bicarbonate, 0.6mM MgCl₂, 0.6mM CaCl₂, 2mM 1-cystein and 25mM PIPES adjusted pH to 7.0). The cell suspension was transferred to a polystyrene tube. To remove large clumps of cell aggregates, the tube sat for 5 minutes at room temperature and then 3mls of the top layer of the cell suspension was carefully removed for the assay.

### Cathepsin B assay

50µl of the cell suspension was added to a black walled 96 well plate (ThermoLabsystems, Part No. 8220, Vantaa, Finland). The appropriate concentration of inhibitors of cathepsin B, purified AHSG or concentrated media were diluted in 5µl of PAB and the added to the cell suspension in each of the 96 wells. The plate was pre-incubated at 40°C for 30 min. The reaction was started by adding 50µl of a 200 µM solution of z-Arg-Arg-AMC (EMD Biosciences Inc, Darmstadt, Germany), a fluorogenic peptide that is lysed by cathhepsin B. This substrate was dissolved in PAB and pre-incubated at 40°C prior to use. Hydrolytic activity of cathepsin B produces free AMC from the substrate peptide resulting in increased fluorescence. The intensity of fluorescence was measured using ThermoLabsystems Fluoroskan microplate reader with a 355nm excitation filter and a 420nm emission filter. The stock solution of z-Arg-Arg-AMC was dissolved in DMSO at the concentration of 10 mM and stored in -80°C. The Cathepsin B specific inhibitors CA-074 and Ac-LVK-CHO (EMD Biosciences Inc., Darmstadt, Germany) were dissolved in H₂O at the concentration of 10mM, aliquoted and stored in -80°C.

### AHSG from AAV-AHSG infected HEK293

HEK 293 cells were subcultured at the cell density of 40% confluence in the morning of the study. After 6 hours of growth, the cells were infected with AAV-AHSG or negative control AAV-lacZ with the M.O.I. of AAV being 3. The next day culture medium was replaced with fresh D-MEM supplemented with 5% FCS and culture for 2 additional days. The culture medium was passed through a filter with the pore size of 0.22 micron and then stored at -80°C. AHSG has a molecular weigh of 55kD. We partially purified and concentrated the spent media that had a molecular mass between 100kD to 30kD. A centrifugal filtration unit, NanoSep 100 (Pall Corporation, Ann Arbor, MI) were applied to collect the molecular weights lower than 100kD. The 0.4ml of the flow through fraction from NanoSep 100 was applied to Nanosep30 and the remaining protein on the filter membrane was re-suspended in 40µl of PAB (Hank's balanced salt solution without sodium bicarbonate, 0.6mM MgCl₂, 0.6mM CaCl₂, 2mM 1-cystein and 25mM PIPES adjusted pH to 7.0) The protein concentration was determined using Bradford reagent (Bio-Rad, Hercules, CA). The aliqouts of protein solution was flash frozen by liquid nitrogen and stored in -80°C prior to use.

Therapeutic approaches are not limited to gene therapy but also include the administration of a protein, small molecule, compound or the like, which provides the desired functional activity by an active component replacement approach to the patient in need.

### Example 1

### Eye

The eye may be considered to be a potentially ideal organ for gene therapy approaches for a number of reasons. Numerous diseases of the eye are now well defined at the molecular level, appropriate animal models are often available, eye morphology and function is simple to determine, the immune privileged nature of the eye is advantageous for gene therapy approaches and the second eye can often be used as a control during the development and testing of therapeutic approach in animal models.

In accordance with the principles and aspects of the present invention there are provided methods to treat disorders of the eye by providing the eye with the ability to downregulate both cathepsin activity and TGF-β receptor binding. This may be achieved, for instance, by providing to the eye, a protein, for example AHSG, having both cathepsin inhibiting activity and TGF-β receptor block the activity. Similarly, peptides and the like which also have the ability to reduce both cathepsin activity and TGF-β activity are considered to be equivalent and are contemplated in the invention.

In a preferred embodiment of the invention, agents of the invention, for example but not limited to, proteins such as AHSG may be provided to the eye in a variety of ways including, for example, topically or intraocularly.

The desired activities of the present invention provided, for example by AHSG, may also be delivered in nucleic acid form, such as through delivery of DNA, RNA, synthetic oligonucleotides or the like. One method particularly contemplated comprises the delivery of DNA coding for a protein, such as, for example, AHSG or a variant thereof. Said DNA may be ideally provided as part of a gene therapy approach, either in a viral vector format or by way of non-viral transfer techniques.

Non-viral nucleic acid transfer techniques include electroporation, transport by gene gun, delivery via liposome encapsulation or simple injection of the naked DNA. Viral nucleic acid transfer techniques can advantageously utilize as vectors a virus selected from any one of several different virus families, including retrovirus (e.g. oncoretrovirus such as Moloney murine leukemia virus; lentivirus such as human or simian immunodeficiency virus, or vesicular stomatitis virus) and adenovirus (e.g. adenovirus; adenoassociated virus; herpes virus such as herpes-simplex virus type 1, cytomegalovirus or Epstein-Barr virus).

A preferred embodiment of the present invention comprises the delivery of the nucleic acid of choice using adenoassociated virus (AAV) as a vector. AAV is particularly useful as a vector for the delivery of nucleic acids to the eye because it possesses: a relative lack of pathogenicity, the ability to induce long-term transgene expression and, it has been shown to transfect a variety of ocular cell types including photoreceptors, retinal pigmented epithelial cells, Muller cells, retinal ganglion cells, trabecular meshwork cells and corneal endothelial cells. The AAV-DNA vector may be injected into the eye at various locations, including the subretinal space or intravitreally.

The AAV-DNA vector may further advantageously comprise various promoters useful to drive expression of the transgene provided to the eye. In particular, preferred embodiments of the invention comprise cytomegalovirus (CMV) and chicken beta-actin (CBA) promoters. Other promoters that may also be used to drive expression in the eye include those associated with platelet-derived growth factor and neuron-specific enolase. In a preferred embodiment, the protein encoding nucleic acid provided to the patient in the adeno-associated virus vector encodes AHSG, or a variant thereof.

The present invention also advantageously provides for treatment of diseases of the eye which are concerned with immune dysfunction, wound healing, scarring and infection. Immune dysfunction related disorders of the eye include autoimmune disorders and transplantation. An autoimmune disorder for which application of the invention is particularly beneficial is autoimmune uveitis, which may be treated both in preventative fashion and as ongoing chronic therapy. Another autoimmune disorder for which the invention is beneficial is dry eye syndrome, which is characterized by inflammation of the lacrimal glands.

Sequelae associated with transplantation are particularly amenable to treatment by the present invention. Tissues to be grafted into the patient may be treated ex *vivo* with a gene therapy approach utilizing nucleic acids of the present invention. Such treatment preferably occurs prior to transplantation in order to render the transplanted tissue less stimulatory to the host's immune system. Alternatively, or in addition, agents of the present invention may be provided directly to the host before, during and/or after transplantation in order to reduce such sequelae. Penetrating keratoplasty (corneal transplantation) is a transplantation procedure that can particularly benefit from the present invention, as the 5-year failure rate due to graft rejection is approximately 50% and the use of toxic, systemic immunosuppression drugs is the current standard of care.

Infectious diseases of the eye may also be beneficially treated by the agents and methods of the present invention. A predominant infectious disease of the eye for which the present invention may be particularly effective is herpes virus type I induced keratitis. This disease is often recurrent and as such would be particularly conducive to the long-term efficacy that comes from nucleic acid delivery of the agents of the invention, which lead to long-term retention of the nucleic acid and consequently to long-term expression of the peptide or protein conferring simultaneous TGF-β activity downregulation and cathepsin activity downregulation

Additionally, wound healing disorders of the eye may also advantageously benefit from treatment with the methods and agents of the present invention. A preferred embodiment of the invention comprises modulating wound healing that follows glaucoma filtration surgery. Because the major portion of wound healing following glaucoma surgery occurs in the two weeks directly following surgery, and because TGF-β activity is the main cytokine involved in the fibroproliferative scarring response in the eye, the agents of the present invention will be ideally provided to the eye before and/or during this time period. Such agents may be provided with or in the absence of other therapeutics, such as mytomycin, 5-fluorouracil and other antimetabolites. Agents of the contemplated invention that may be provided to glaucoma surgery patients include but are not limited to nucleic acids that code for AHSG or other similarly acting polypeptides, or peptides or proteins that advantageously provide cathepsin downregulating activity and TGF-β inhibiting activity. Such nucleic acids may be advantageously provided directly to the eye, in either viral encapsulated or naked formats, or to other cells ex vivo which are then subsequently introduced to the eye.

Another wound healing disorder of the eye that can benefit from treatment with the methods and agents of the present invention is proliferative vitreoretinopathy (PVR). PVR is the major cause of failure of retinal reattachment surgery and is associated with the deposition of extracellular matrix proteins and the formation of fibrovascular membranes, events which are mediated in part by the activities of TGF-β. Agents of the present invention that may be provided to retinal reattachment patients in order to prevent or treat PVR include but are not limited to nucleic acids that code for AHSG or other polypeptides, or peptides or proteins that provide cathepsin downregulating activity and TGF-β downregulating activity. When nucleic acids are provided to the eye, in either viral, encapsulated or naked formats, the nucleic acids may be advantageously provided directly to the eye, or they may be first applied to other cells ex vivo which are then subsequently introduced to the eye.

### Example 2

### Liver

TGF-β is central to the progression of liver disorders involving inflammation and/or fibrosis and fibrogenesis. Decreasing TGF-β activity and cathepsin activity therefore represents a highly beneficial therapeutic approach. The various agents and methods contemplated by the present invention are useful in the treatment of liver disorders, including toxic, cholestatic, alcoholic (e.g. cirrhosis), inflammatory disorders as well as other types of liver injury such as biliary atresis.

One embodiment of the present invention comprises providing therapeutic agents of the present invention to the liver in order to reduce the biological activity of TGF-β and cathepsin. Such agents may be advantageously delivered as polypeptides or in nucleic acid form, which may be provided by a viral or non-viral vector and which may be provided directly to the patient or alternatively to other cells which are then provided to the patient. In a preferred embodiment, when the agent or agents of the invention are provided directly to the patient and intended for the liver, they are administered by way of the portal vein. In another preferred embodiment, the agents may be provided to the patient by injection into the patient's circulation.

The liver is itself an excellent target for in vivo delivery of genes because it is easily accessible via large pores associated with the liver's capillaries, which themselves may be conveniently accessed via injection of the agents into the circulation of the patient. Suitable gene delivery vehicles may comprise liposomes, DNA (or RNA)-protein complexes, viral vectors, cells (for example, hepatocytes) containing the transgene, naked DNA, RNA or the like. In a preferred embodiment of the invention, the nucleic acid delivered is a DNA. In a most preferred embodiment, the nucleic acid is delivered by a viral vector. In another preferred embodiment, the nucleic acid codes for AHSG or another polypeptide that provides both cathepsin and TGF-β downregulating activities. In another especially preferred embodiment, the viral vector comprises adeno-associated virus and most preferably further comprises a promoter driving the expression of the nucleic acid selected from promoters associated with the albumin gene, the immediate early cytomegalovirus gene, the human phosphoglycerate kinase gene, the 5'-LTR of the Moloney murine leukemia virus and the chicken β-actin gene.

### Example 3

### Kidney

Extracellular matrix accumulation in the glomeruli is common to and the major cause of the pathogenesis of essentially all progressive renal diseases that lead to end-stage renal failure. Currently, no specific and effective therapy is available to treat or prevent the progression of renal fibrosis. TGF-β plays an integral role in the progression of renal fibrotic diseases, for example glomerulonephritis and diabetic nephropathy. While not wishing to be bound by any particular theory, we believe that downregulation of TGF-β receptor binding and cathepsin activity will decrease fibrotic deposition and inhibit the subsequent progression of renal disease.

A therapeutic treatment embodiment of the present invention comprises providing agents in therapeutic effective amounts to the kidney in order to reduce the activities of TGF-β and cathepsin therein. As with treatment of the liver, such agents may be advantageously delivered as polypeptides or in nucleic acid form, which may or may not be carried by a viral or non-viral vector, applied either directly to the patient or to other cells which are then provided to the patient.

For example AHSG may be ideally provided to the patient to treat or prevent fibrotic renal disease. In another embodiment of the invention, a therapeutically effective amount of a polypeptide that provides TGF-β downregulating and cathepsin downregulating activity may be provided to the patient. In another embodiment of the invention the AHSG or polypeptide that provides TGF-β downregulating and cathepsin downregulating activity may be provided by intravenous injection. In another embodiment of the invention, the AHSG or polypeptide that provides TGF-β downregulating and cathepsin downregulating activity may be provided to the patient by introduction of a nucleic acid directly to the patient. This nucleic acid, which may be RNA or DNA, will ideally code for AHSG or other polypeptide with comparable activity whereby expression of the protein in the living patient would result in the therapeutically beneficial activity of the AHSG or polypeptide being provided to the patient. In another embodiment of the invention, the nucleic acid may be administered to a population of mammalian (preferably human) cells outside of the body, and these transformed cells would then subsequently be provided to the patient where they would then express the AHSG or other polypeptide and downregulate TGF-β and cathepsin, thereby resulting in treatment of the patient. In a preferred embodiment, the nucleic acid would be DNA. In another preferred embodiment, the nucleic acid would be delivered to the cells, either in the body or outside the body, through use of a viral vector. In a still more preferred embodiment, the viral vector would comprise adeno-associated virus. In another preferred embodiment, the delivery of the nucleic acid may be by injection into the circulation, directly into the kidney, or into the renal parenchyma. In another preferred embodiment, the delivery of the nucleic acid in the AAV viral vector may be advantageously enhanced by addition of an enhancing agent. Such an enhancing agent may be selected from a list of agents that (1) damage DNA (such as cisplatinum or ionizing radiation, (2) deplete cellular nucleotide pools (such as hydroxyurea, (3) inhibit topoisomerase activity (such as etoposide or camptothecin) or (4) arrest the cell cycle (such as aphidicolin).

### Example 4

### Pulmonary

Overexpression of TGF-β is the primary cause and contributor to the extracellular matrix deposition that characterizes the pathogenesis of pulmonary fibrosis. The steroids and other immunesuppressive agents that are conventionally used for idiopathic pulmonary fibrosis are less than ideally effective, generally resulting in less than 50% survival at five years. The present invention contemplates methods and agents which advantageously downregulate TGF-β activity and cathepsin activity and thus provide improved treatment and prevention of pulmonary fibrosis.

One contemplated embodiment of the present invention comprises delivery of a protein, with the ability to downregulate TGF-β activity and cathepsin activity, to the lungs in order to treat or prevent pulmonary fibrotic diseases. The protein may be delivered by various approaches, for example as a protein, as a nucleic acid which codes for the protein upon expression, or as a cell carrying the nucleic acid which codes for the protein. The nucleic acid may be advantageously delivered either as naked DNA or carried in a vector. Such a vector may comprise for example a virus, such as adeno associated virus or AAV. Delivery of the protein, in whatever form, may occur by intratracheal, intravenous or intratracheal routes for example. When delivered by intravenous or intratracheal routes, the delivery may be for example by injection. When delivered by intravenous routes, the protein of interest may be found to accumulate in the liver, lungs and kidneys making intravenous administration especially efficient for treating a number of fibrotic disorders associated with these organs. When delivered by an intratracheal or nasal route, the delivery may advantageously be formulated in aerosol form for delivery with aerosol devices pursuant to methods well known to those skilled in the art.

### Example 5

### Myocardial

TGF-β is a major stimulator of fibroblast proliferation, phenotypic conversion of fibroblasts to myofibroblasts and extracellular matrix production in the heart. Accumulation of such fibrous tissue in the heart leads to impaired stiffness and pumping capacity in hypertrophied hearts, as well as a variety of ventricular dysfunctions, and ultimately diastolic dysfunction and heart failure. Until this invention, blocking TGF-β stimulation by down-regulating TGF-β activity and by reducing cathepsin activity has not been available.

A preferred embodiment of the present invention comprises delivering protein having the ability to downregulate both TGF-β activity and cathepsin activity to the heart in order to treat or prevent cardiovascular fibrotic diseases, such as for example diastolic dysfunction. The protein of the present invention may be delivered by intravenous, intramuscular or intraperitoneal, or other injection routes, or may be injected directly into the heart tissue or into nearby cellular tissue. In another embodiment of the invention, the protein may be delivered in the form of a nucleic acid, which may be DNA or RNA, which may be naked or carried in either a viral or non-viral vector. In a most preferred embodiment of the present invention, the protein comprises AHSG, and is provided directly by injection or its encoding gene is similarly provided in an AAV vector or an adenoviral vector.

### Example 6

### Ocular Applications and Procedures for Selecting Compounds Active for Ocular Application and for Identifying Proper Doses

### Use of Rabbit Model of Proliferative Vitreoretinopathy (PVR)

In accordance with conventional techniques, experimental PVR is induced in rabbits, putative therapeutic agent,-injected, and the development of PVR is monitored to determine the level of effectiveness of the therapeutic agent. The desired therapeutic agent may be a chemical entity such as a protein or peptide with TGF-β receptor blockade activity and cathepsin inhibiting activity. An example of such a protein is AHSG. The therapeutic agent may also be delivered via its encoding nucleic acid provided that the expressed protein or peptide demonstrates TGF-β receptor blockade activity and cathepsin inhibiting activity. In accordance with well known techniques, the nucleic acid may be administered unencapsulated, carried by a viral vector, carried by a transfection enhancing agent such as a liposome, or carried in a cell which is itself delivered to the patient. Suitable viral vectors include adeno-associated virus, or any other virus or viral components known to be useful for the delivery of coding sequences to cells of interest. The genetic techniques for identifying the gene encoding the proteins or peptides shown to have anti-PVR activity are well-known as are the techniques for splicing them into suitable viral vectors and need not be received here. These aspects apply to all of the testing models described herein.

The rabbit model of PVR is described by Oshima (Oshima et al Gene Ther 9:1214 (2002)), fully incorporated herein by reference) where it was used to demonstrate that reduction of TGF-β expression in the eye results in reduced clinical markers of PVR. It is proposed by the authors that an agent (specifically a gene delivered by a viral vector, in this paper) which reduces the clinical markers of PVR in the eye of a test rabbit is a model for (gene) therapy for the treatment of PVR in the human eye.

In brief synopsis, in Oshima's rabbit PVR model, adult rabbits are anesthetized with an intramuscular injection of ketamine hydrochloride (14 mg/kg) and xylazine hydrochloride (14 mg/kg). The pupils are dilated with a drop of 10% phenylephrine hydrochloride, 1% tropicamide, and 1% atropine sulfate. One eye of each rabbit is injected with 500,000 fibroblasts in 0.1 mL BSS solution in the vitreous cavity through the pars plana, following pars plana vitrectomy. Immediately thereafter, the eyes receive a single intravitreal injection of the therapeutic agent to be screened on whose dosing is to be identified through standard activity titrating techniques. A similar number of eyes receive only fibroblasts and balanced salts solution as a control. All eyes are ophthalmoscopically examined daily up to 1 month following surgery and the development of PVR is classified using Fastenberg clinical criteria.

The choice of viral vector to carry a gene into the eye has significant impact on the outcome of the therapy. Recombinant adeno-associated virus provides several key advantages over other viral vectors for delivery to the eye, especially a relative lack of pathogenicity and the ability to induce long-term transgene expression (reviewed in Martin, K.R. et al Methods 28:267 (2002)). Investigation of the six subtypes of AAV has demonstrated the greatest expression in the eye is achieved using subtypes AAV4 and AAV5 (Rabinowitz, J.E. et al J Virol 76:791 (2002))

An effective dose for the rabbit eye of a viral-vector-carried gene coding for a soluble TGF-β receptor, which will be similar in effectiveness to AHSG because of the ability to block binding between TGF-β and its receptor, was found by Oshima to be 0.1 ml of 10³ PFU. Drawing conclusions from the work of these authors an appropriate starting dosage to treat a human eye would be 10⁴ PFU delivered by direct intravitreal injection (Oshima, Y, et al Gene Ther 9:1214 (2002)) in 0.1 ml of diluent (preferably sterile saline solution), as a rabbit's eye is approximately 1/10th the mass of a human eye. However, AAV viral titer research in the eye has demonstrated that higher titers provide better efficiency of gene transfer, with the best results in a mouse eye obtained with 10⁷ PFU provided at a concentration of 10¹⁰ PFU per ml (Sarra, G.M. et al Vision Res 42:541 (2002)). This implies optimal gene transduction efficiency is more likely to be achieved with doses of approximately 10⁹ PFU at a concentration of 10¹⁰ PFU per ml in 0.1 ml, given that the human eye is approximately 100 times the size of a mouse eye. Thus the starting range from which to optimize the human dosage would be 10⁴ to 10⁹ PFU, with improved transduction efficiencies and thus greater therapeutic effects to be expected from the higher end of the range. The virus would be intravitreally injected initially once per month and the need for repeat injections ascertained by examining the progression of PVR using Fastenberg's clinical criteria (Fastenberg, D.M. et al Am J Ophthalmol 93:565 (1982)). A detailed description of the method for intravitreal injection is disclosed by Martin and coworkers (Martin, K.R. et al Methods 28:267 (2002)) and is fully incorporated herein by reference. Any progression of the disease on the Fastenberg scale will indicate the need for a repeat administration of the therapy.

### Use of Primate Model of Ocular Hypertension

Another useful screening and dose titrating techniques involves a primate model wherein Ocular hypertension is induced in monkeys following which glaucoma trabeculotomy surgery takes place and the therapeutic agent to be identified or whose therapeutic dose is to be identified is delivered. The wound healing process is then monitored to determine the level of effectiveness of the therapeutic agent. The described therapeutic agent will demonstrate TGF-β receptor blockade activity and cathepsin inhibiting activity, such as demonstrable by AHSG.

The primate model of ocular hypertension was described by Heatley and coworkers (Heatley, G. et al Gene Therapy 11:949 (2004)) at which time it is proposed to act as an accurate model of the fibrotic wound healing disorders that follow glaucoma surgery in the human. Successful therapy in this primate model is believed to correlate with successful therapeutic intervention for human patients. Expected optimal dosage range (i.e. PFU and concentration), viral vector choice, and preferred route of administration in the human patient is (as described above for the rabbit model, rather than as described in the primate model of the human disease) Standard clinical methods to examine intraocular pressure (IOP) are utilized, including Goldman applanation tonometry, which is known to be applicable to both monkey and human eyes (Kaufman, P.L. et al Arch Opthalmol 98:542 (1980)). Methods to examine positive outcome for post-operative scarring following glaucoma surgery, and discussion linking those methods to positive outcome in human patients, is disclosed by Mead et al (Mead, A.L. et al Invest Ophthalmol Vis Sci 44:3394 (2003)) and human clinical trial criteria are disclosed by Cordeiro et al (Cordeiro, M.F. et al Invest Ophthalmol Vis Sci 40:2225 (1999)). Following treatment of the human patient as described and examination of clinical success using standard criteria, progression of scarring or increasing IOP will indicate the need for repeat administration of the therapy.

In the Heatley model, Cynomologous monkeys are anesthetized with ketamine hydrochloride (10 mg/kg) and a modified goniolens is placed on one eye. A laser is used to ablate 180 degrees of the trabecular meshwork and intraocular pressure (IOP) is monitored for elevation daily. Animals receive trabeculotomy surgery once they exhibit significantly reduced outflow facility and have a sustained elevation of IOP. For the surgery, the animals are anesthetized with intramuscular ketamine followed by intravenous sodium pentobarbital (15 mg/kg) and conventional trabeculotomy is performed in the superior quadrant. Following incision of the conjunctive and Tenon's capsule approximately 8 mm from the superior corneal limbus, lysis of densely adherent Tenon's capsule is performed to the limit of the limbus and the scleral bed is thoroughly cauterized. Weck-Cel sponge soaked with the therapeutic agent or a control agent is placed in the subconjuntival space for 5 minutes. The sponge is removed and the area rinsed with BSS and a 2x2 mm half-thickness scleral trabeculectomy flap is created. A Kelly Descemet punch is used to create a wide ostomy, and a partial iridectomy is performed. The scleral flap is closed with two nylon sutures at each corner. Flow through the flap is ascertained by inflating the anterior chamber with NSS through a limbal paracentesis, and the conjunctival incision is closed in a single layer with a running Vicryl® suture. Antibiotic and steroid is applied topically after surgery. Following surgery animals are evaluated with slit-lamp examinations scoring the anterior chamber for cells and flare, clarity of the cornea and lens, inflammation of the conjunctiva, status of the iris and fistula, and the morphology of the surgical bleb. IOP is measured by minifield Goldman applanation tonometry using a Haag-Streit slit lamp.

### Example 7

### Hepatic Applications

### Use of a Rat Model of Liver Fibrosis

The dimethylnitrosamine rat model of persistent liver fibrosis has been described as an established model that closely resembles the pathophysiology of human liver cirrhosis (Jenkins, S.A. et al J Hepatol 1:489 (1985); Jezequel, A.M. et al J Hepatol 5:174 (1987), both fully incorporated herein by reference). Successful therapeutic intervention in the progression of this experimental disorder in rats is expected to correlate to a successful therapeutic intervention modality for use in human persistent liver fibrosis disorders, such as cirrhosis (Qi, Z. et al Proc Nat Acad Sci 96:2345 (1999)).

In accordance with this well known animal model, liver fibrosis is induced in rats and the therapeutic agent to be screened or dosage determined is delivered. Liver fibrosis is then examined to determine the level of effectiveness of the therapeutic agent. In this animal model, Male Sprague-Dawley rats approximately 10 weeks old and weighing around 350 grams are given a single infusion of 1 ml of either the therapeutic agent or saline via the portal vein. Alternatively, the therapeutic agent may be provided via intramuscular injection. Each rat then receives an intraperitoneal injection of dimethylnitrosamine (DMN, 10 microgram/gram) three times per week for 3 weeks which initiates irreversible liver fibrosis. At the end of the 3-week period, venous blood is collected and the rats are sacrificed. Biochemical parameters, such as hyaluronate, aspartate aminotransferase and alanine aminotransferase, are measured using standard methods. The liver is fixed with 4% paraformaldehyde for histological examination. Tissue sections are stained with Masson-trichrome or subjected to immunohistostaining using antibodies against collagen Type-I, fibronectin, alpha-smooth muscle actin, TGF-β, or monocytes/macrophages. Immunoreactive materials are visualized using biotinylated antimouse (or anti-rabbit) IgG antibody, peroxidase-labeled stireptavidin and diaminobenzidine. For the semi quantitative analysis of fibrosis, the blue-stained area in the Masson-trichrome stained sections may be measured on a video-screen display using a digital image analyzer.

The choice of the *in vivo* therapeutic gene delivery modality as a method of treatment for liver fibrotic disease in humans is proposed because the liver is an excellent target, being easily accessible to vectors injected in the circulation through large pores in liver capillaries (Fraser, R. et al Hepatology 3:863 (1997)). Adeno-associated virus is likely to be the best means of delivery of a therapeutic gene to human liver cells, because it displays efficient, stable expression and invokes minimal immune response, in contrast to nonviral gene delivery or adenoviral vector deliver (reviewed in Xiao, W. et al J Virol 72:10222 (1998)). A highly effective promoter for in vivo expression in liver is the long terminal repeat (LTR) promoter or the albumin promoter (Snyder, R. O. et al Nat Genet 16:270 (1997); Xiao, W. et al J Virol 72:10222 (1998)). Dosage of virus for human therapeutic use is expected to be optimal at approximately 5x10¹⁴ infectious units, based upon the observed dose response curve for mice, the optimal mouse dosage for efficient gene transduction of 10¹¹ infectious units and the known dose:dose conversion of mice to humans (Xiao, W. et al J Virol 72:10222 (1998)). Human administration of the viral vector carrying the transgene is straightforward, requiring only infusion into the portal circulation (Snyder, R.O. et al Nat Genet 16:270 (1997); Xiao, W. et al J Virol 72:10222 (1998)). Several methods are available for examining the effectiveness of the therapeutic administration. Liver specimens may be obtained by biopsy from patients and histologically graded for fibrosis using Masson's trichrome staining (Ferrell, L. Mod Pathol 13:679 (2000)). The lack of fibrosis demonstrates the effectiveness of the therapy, while progression of fibrosis indicates the need for additional administrations and/or higher dosages of the therapeutic agent.

### Example 8

### Renal Applications

### Use of a Rat Model of Glomerulonephritis

The anti-thymocyte serum injection induced rat model of glomerulonephritis has been described by Okuda as reflecting human kidney fibrosis (Okuda, S. et al J Clin Invest 86:453 (1990), fully incorporated herein by reference). Therapeutic regimes that result in reduced fibrosis in this rat model are expected to also be effective when used therapeutically in human fibrotic kidney disorders (Border, W.A. et al Nature 361:361 (1992), fully incorporated herein by reference).

In the Oduda animal model, Glomerulonephritis is induced in rats following which the therapeutic agent to be screened or dosage determined is delivered. Progression of fibrotic renal disease is then examined to determine the level of effectiveness of the therapeutic agent. Glomerulonephritis is induced in 4-6 week old Sprague-Dawley rats by intravenous injection of antithymocyte serum. One hour after antithymocyte injection, and daily for 6 days, the rats are administered a 0.5 ml intravenous injection of the therapeutic agent or phosphate buffered saline as a control. On day 7, urine is collected from all rats, and urinary protein and creatinine is measured. The animals are sacrificed and fibronectin in glomeruli quantified to assess the ability of the therapeutic agent to block matrix deposition in Glomerulonephritis. The glomeruli may also be stained with antibodies to extra domain A of fibronectin and tenascin to detect TGF-β activity or analyzed histologically with acid-Schiff staining. Urinary protein to urinary creatinine ratios will indicate the development of proteinuria.

When the therapeutic agent to be delivered to the animals is provided in the context of a viral vector, such as adenoassociated virus, the agent may alternatively be delivered directly to the kidneys by exposing the kidneys with flank incisions and injecting each kidney at several sites with the virus in phosphate buffered saline.

Gene therapy as a method of treating renal disease in humans requires careful consideration of the delivery system. While liposomal-complexed DNA can be expressed in renal cells in vivo, the expression is transient and modest (Tomita, N. et al Biochem Biophys Res Commun 186:129 (1992); Isaka, Y. et al J Clin Invest 92:2597 (1993); reviewed in Lien, Y.H. et al Kidney Int 52:S85 (1997); all of which is-fully incorporated herein by reference). Retroviral vectors result in inefficient transduction of the kidney because only dividing cells are easily transduced (Bosch, R. J. et al Exp Nephrol 1:49 (1993)), and adenoviral vectors result in expression lasting only a few weeks (Moullier. P. et al Kidney Int 45:1220 (1994)). Recombinant adeno-associated virus is the most attractive vector for treating human renal disease, as AAV does not activate cell-mediated immunity, is able to transduce both dividing and non-dividing cells, drive long term expression and are replication deficient in the absence of a coinfecting helper virus (reviewed in Lipkowitz, M.S. et al Am J Kidney Dis 28:475 (1996)). Appropriate dosage for a human may be estimated from the effective transducing dose of AAV to the kidney of a mouse. Mice directly injected in the kidney were efficiently transduced with 106 infectious units in 80 µl phosphate buffered saline per kidney and an appropriate starting dosage for humans could therefore be estimated at approximately 10¹⁰ infectious units per human kidney when administered by direct injection (Lipkowitz, M.S. et al. J Am Soc Nephrol 10:1908 (1999)). The route of administration for humans may be best served by either kidney artery perfusion (Daniel, C. et al Am J Pathol 163:1185 (2003) or direct injection at several locations in the kidney itself (Lipkowitz, M.S. et al J Am Soc Nephrol 10:1908 (1999)). To determine effectiveness of therapy and the need for additional administrations, fibronectin may be monitored using standard clinical methods as fibronectin is greatly increased in human mesangial proliferative glomerulonephritis (Oomura, A. et al Virchows Archs A Pathol Anat Histopatho 415:151(1989); Courtoy, P.J. et al. J Cell Biol 87:691 (1980); Courtoy, P.J. et al J Histochem Cytochem 30:874 (1982); Border, W.A. Kidney Int 34:419 (1988)). Stable fibronectin indicates effective human therapy, while increasing fibronectin levels indicates progression of the disease.

### Example 9

### Pulmonary Applications

### Use of a Mouse Model of Pulmonary Fibrosis

The mouse model of pulmonary fibrosis has been disclosed by Shimizukawa (Shimizukawa, M. et al Am J Physiol Lung Cell Mol Physiol 284:L526 (2003); Swiderski, R.E. et al Am J Pathol 152:821 (1998), both fully incorporated herein by reference) and the model is considered to mimic the symptoms and pathogenesis of human idiopathic pulmonary fibrosis. Successful therapeutic intervention in this mouse model is proposed to correlate to a successful therapeutic intervention modality for use in human pulmonary fibrosis (Swiderski, R.E. et al Am J Pathol 152:821 (1998); Onuma, K. et al Tohoku J Exp Med 194:147 (2001), both fully incorporated herein by reference).

In accordance with this conventional model, Pulmonary fibrosis is induced in mice following which the therapeutic agent is delivered. Progression of pulmonary fibrosis is then examined to determine the level of effectiveness of the therapeutic agent. Pulmonary fibrosis is induced in 12 week old female C57BL/6 mice by intraperitoneal instillation of 0.75 mg bleomycin chlorate in 0.1 ml saline every other day for 7 days. The therapeutic agent is delivered via an intratracheal route injected directly into the trachea under 2.5% tribromoethanol anesthesia or via an intravenous route injected into the jugular vein through a 29-gauge needle. One month later the animals are sacrificed and the lungs infused through the trachea with 10% buffered formalin and fixed at room temperature for 16 hours. Lungs are analyzed for hydroxyproline content via HPLC or embedded in paraffin and hematoxylin, eosin and elastic-Masson stained sections are prepared. Hydroxyproline content is found to be reduced relative to positive controls in animals treated with an effective therapeutic agent, and histological analysis shows reduced fibroproliferation and microhoneycomb-like lesions in the subpleural regions of animals treated with an effective therapeutic agent relative to positive control animals.

While intravenous administration of viral vector carrying DNA has been demonstrated to cause expression in the lung (Border, W.A. et al Nature 360:361 (1992), substantial expression has only been demonstrated by intratracheal administration (Shimizukawa, M. et al Am J.Physiol Lung Cell Mol Physiol 284:L526 (2003)). Direct tracheal injection under anesthesia of virus dissolved in sterile saline is expected to provide the best results, as this is the method of administration demonstrated to be effective in a mouse model of viral gene delivery to the lungs. As discussed previously, recombinant adeno-associated virus is the most attractive vector for treating human disease, as AAV does not activate cell-mediated immunity, is able to transduce both dividing and non-dividing cells, drive-long term expression and is replication deficient in the absence of a co-infecting helper virus. The appropriate human dosage to achieve therapeutically effective expression of the desired protein in the lungs of the human patient may be estimated from the optimal dosage in the mouse, which is found to be 10⁹ infective units in 30 µl sterile saline when administered by intratracheal route (Shimizukawa, M. et al Am J Physiol Lung Cell Mol Physiol 284:L526 (2003)). As human lungs are approximately 5000 times the mass of mouse lungs, an appropriate starting dosage is about 5x10¹² infective units, administered by direct intratracheal injection. To determine the effectiveness of the human therapy, the human lungs may be subjected to X-ray or CAT scan to determine abnormal scarring, and the preferred visualization protocol for the measurement of pulmonary fibrosis disease progression in humans is HRCT (Muller, N.L. et al Radiology 165:731 (1987)). Stabilization of disease indicates effective therapy, while progression indicates the need for additional administration of the agent.

### Example 10

### Cardiovascular Applications: Use of a Rat Model of Myocardial Fibrosis

The rat model of myocardial fibrosis has been described by Kuwahara and coworkers (Kuwahara, F. et al Circulation 106:130 (2002), fully incorporated herein by reference) and is thought to reflect human diastolic heart dysfunction and ultimately failure. Successful therapy in this rat model is believed to indicate a successful therapeutic intervention for human patients. In this model, Myocardial fibrosis is induced in rats and the therapeutic agent is delivered. Progression of myocardial fibrosis is then examined to determine the level of effectiveness of the therapeutic agent to be screened or dosage determined. Mean arterial pressure elevation is induced by suprarenal abdominal aortic constriction of male 8 week old Wistar rats under pentobarbital (50 mg/kg intraperitoneal) anesthesia. Left ventricle hypertrophy will increase progressively in the test animals after day 7 following aortic constriction. Rats are provided with the therapeutic agent by injection intravenously, into skeletal muscle, or by intracoronary administration. Myocardial fibrosis, induction of type I and III collagens, and proliferation of fibroblasts and myofibroblasts is observed in animals not receiving an effective therapeutic agent but is not observed in animals receiving an effective therapeutic agent. Echocardiographic and hemodynamic studies may further be used to examine the effectiveness of a therapeutic agent.

The delivery of genes to adult myocytes in vivo has been demonstrated by injection of plasmid DNA but is limited by low efficiency of transduction and expression (Lin, H. et al Circulation 82:2217 (1990)). Viral delivery of DNA, by adenovirus, has been demonstrated successfully in rodents, and at 10-50 fold higher levels of expression which last up to 1 month, but by then at lower levels (Guzman, R.J. et al. Circ Res 73:1202 (1993); Kass-Eisler, A. et al Proc Natl Acad Sci 90:11498 (1993)). The preferred method of delivering genes to the human heart as a therapeutic modality, derived from successful demonstration in a rodent heart, is direct intracoronary administration (Guzman, R.J. Circ Res 73:1202 (1993)). Adeno-associated virus is the preferred delivery vector, because it results in long-term expression and does not cause a significant cell-mediated immune response (Chirmule, N. et al Hum Gen Ther 10:259 (1999); Chu, D. et al J Thor Card Surg 126:671 (2003)). Delivery of virus has into the heart has been demonstrated by direct intramuscular injection and by infusion of virus into coronary arteries (Muhlhauser, J. et al Gene Ther 3:145 (1996)). Viral delivery of DNA to humans has been achieved with direct myocardial injections as follows: 10 injections are made into sites 1-1.5 cm apart in the diseased area of the myocardium, each injection consisting of 4x10⁸ to 4x10¹² infectious units dissolved in 100µl sterile saline (Rosengart, T.K. et al Circulation 100:468 (1999)). The preferred method of treatment is therefore as described by Rosengart, and utilizing AAV vector carrying our gene of interest (for example that which encodes AHSG). Determination of success of the therapy is by the usual human clinical markers of myocardial fibrosis as is conventionally understood.

### Example 11

### AHSG reduces proliferation, of cancer cells when provided as a therapy.

An appropriate model for determining the ability of a therapy to inhibit the growth of cancer cells is to provide the therapy to be tested to ∈57BL mice which have received subcutaneously injected Lewis Lung carcinoma (LLC) cells and then to measure the growth of resulting tumors over time (Kimura, Y. et al Planta Med 70:211 (2004)). A therapy that inhibits tumor growth when provided to the C57BL mice subcutaneously injected with LLC cells is considered to be a promising therapy for the treatment of human cancer patients.

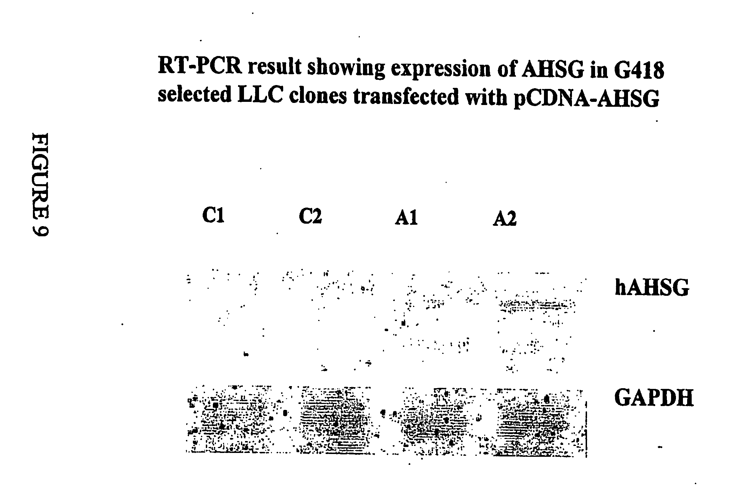

AHSG DNA was provided to LLC cells by transfection using the commercially available peDNA3 vector. Construction of the pcDNA3 vector containing AHSG (pcDNA-AHSG) was performed using standard recombinant DNA' techniques. Superfect reagent was used to improve the efficiency of transfection into the LLC cells according to the manufacturer's protocol (Qiagen NV, KJ Venlo, The Netherlands). As a control, pcDNA3 vector containing no AHSG DNA was transfected into a control culture of LLC cells. Transfected LLC cells were selected using G418 at a concentration of 600 µg/mL for two weeks, following which clones were picked, expanded in culture and stored in liquid nitrogen in the presence of 5% DMSO. Two clones (C1 and C2) of LLC cells transfected with pcDNA3 were isolated and two clones (A1 and A2) of - LLC cells transfected with pcDNA-AHSG were also isolated. The A1, A2, C1 and C2 clones were analyzed for the expression of AHSG by RT-PCR followed by gel electrophoresis as shown in Figure 9. To ensure consistent RNA isolation from the transfected LLC cells, RT-PCR of glyceraldehyde-3-phosphate dehydrogenase (GAPDH) was also performed.

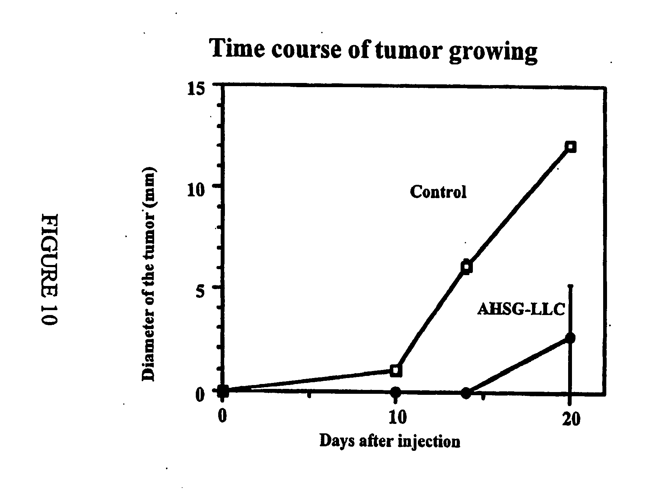

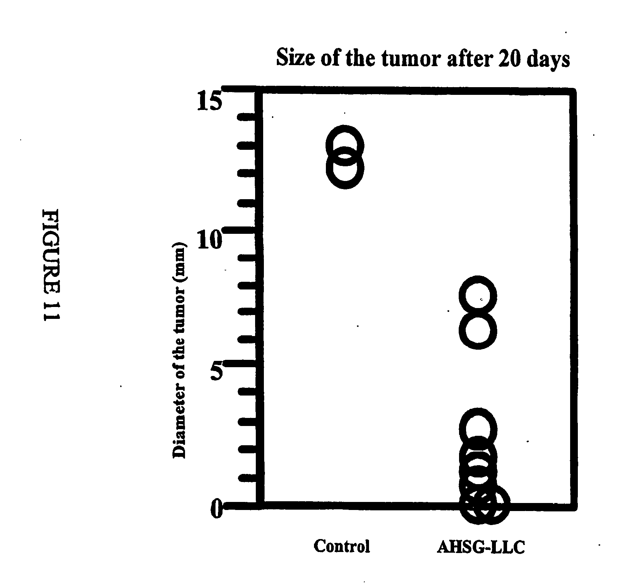

5x10⁴ LLC cells transfected with pcDNA3-AHSG (clone A1) were suspended in 100µl of D-MEM and injected subcutaneously into the posterior region of eight C57BL mice. As a control, 5x10⁴ LLC cells transfected with pcDNA3 not containing AHSG (clone C1) were suspended in 100µl of D-MEM and injected subcutaneously into the posterior region of two C57BL mice. Progression of tumor growth at the site of the injection was determined at days 0, 10, 14 and 20 using calipers. Average tumor sizes at day 0, 10, 14, and 20 are shown in Fig 10. The average tumor size of the control mice (containing pcDNA3 but not the AHSG gene) at day 20 was 12.15 mmn +/- 0.15 mm as shown in Fig. 11. The average tumor size of the mice that received LLC cells transfected with pcDNA-AHSG at day 20 was 2.6 mm +/- 2.6 mm as shown in Fig. 11. These results demonstrate that AHSG is therapeutically useful for the treatment of cancer.

Although the above disclosure describes and illustrates various embodiments of the present invention it is to be understood that the invention is not to be limited to these particular embodiments. For full definition of the scope of the invention, reference is to be made to the appended claims.

## Claims

1. Lymphocytes transformed with a nucleic acid encoding α2-Heremans-Schmid glycoprotein for use in the treatment of a patient suffering from cancer.

2. The lymphocytes of claim 1, wherein said lymphocytes are isolated from at least one tumor bearing animal.

3. The lymphocytes of claim 2, wherein said tumor bearing animal is the same as said patient.

4. The lymphocytes of any one of claims 1 to 3, wherein said nucleic acid is introduced through use of an adeno-associated viral vector.

5. The lymphocytes of anyone of claims 1 to 4, wherein said lymphocytes are comprised in a pharmaceutical composition which is to be administered by injecting said transformed lymphocytes intravenously, intrahumorally, intramuscularly or subcutaneously.

6. The lymphocytes of anyone of claims 1 to 5, wherein said lymphocytes are enriched for CD8 expressing lymphocytes.

7. The lymphocytes of anyone of claims 1 to 6, wherein said transformed lymphocytes are suspended in a pharmaceutically acceptable carrier.

8. An in vitro method for enhancing the immunological capacity of lymphocytes comprising transforming said lymphocytes with a nucleic acid encoding a2-Heremans-Schmid glycoprotein.

9. The method of claim 8 or the lymphocytes of claim 1, wherein said lymphocytes are previously isolated from an animal and to be reintroduced in transformed form to the same animal or another animal.

10. Lymphocytes which are transformed with a nucleic acid encoding α2-Heremans-Schmid glycoprotein, wherein α2-Heremans-Schmid glycoprotein blocks TGF-β by inhibiting the activity of cathepsin B and blocking binding of TGF-β with the TGF-β receptor, for use in treating a patient suffering from disease or medical condition associated with the presence of TGF-β, wherein said disease or medical condition is selected from cancer, transplantation, autoimmune disorders, wound healing, scarring, and inflammation which is affecting one or more tissues of said patient.

11. The lymphocytes of claim 10, wherein one or more tissues is/are selected from the group consisting of eye, kidney, liver, glands, cardiovascular, pulmonary, and myocardial.

12. The lymphocytes of claim 10 or 11, wherein said lymphocytes block TGF-β by competitive binding of the α2-Heremans-Schmid glycoprotein to the TGF-β receptor.

13. The lymphocytes of any one of claims 10 to 12, wherein the lymphocytes were transformed by in vivo gene therapy.

14. The lymphocytes of any one of claims 10 to 12, wherein the lymphocytes were transformed by ex vivo gene therapy.

15. The lymphocytes of anyone of claims 10 to 14, wherein the lymphocytes are comprised in a pharmaceutical composition which further comprises a pharmaceutically acceptable carrier.

16. The lymphocytes of anyone of claims 10 to 15, wherein the nucleic acid is presented in a viral vector.

17. The lymphocytes of claim 16, wherein the viral vector comprises an adenovirus associated viral vector.

18. An isolated lymphocyte transformed with a nucleic acid encoding α2-Heremans-Schmid glycoprotein.

## Patentansprüche

1. Lymphozyten, die mit einer α2-Heremans-Schmid-Glykoprotein kodierenden Nukleinsäure transformiert sind, für die Verwendung in der Behandlung eines Patienten, der an Krebs leidet.

2. Lymphozyten nach Anspruch 1, wobei die Lymphozyten von mindestens einem tumortragenden Tier isoliert sind.

3. Lymphozyten nach Anspruch 2, wobei das tumortragende Tier das gleiche ist wie der Patient.

4. Lymphozyten nach einem der Ansprüche 1 bis 3, wobei die Nukleinsäure unter Verwendung eines adenoassoziierten viralen Vektors eingebracht wird.

5. Lymphozyten nach einem der Ansprüche 1 bis 4, wobei die Lymphozyten in einer pharmazeutischen Zusammensetzung enthalten sind, die mittels Injizieren der transformierten Lymphozyten auf intravenösen, intrahumoralen, intramuskulären oder subkutanem Weg zu verabreichen ist.

6. Lymphozyten nach einem der Ansprüche 1 bis 5, wobei die Lymphozyten hinsichtlich CD8-exprimierender Lymphozyten angereichert sind.

7. Lymphozyten nach einem der Ansprüche 1 bis 6, wobei die transformierten Lymphozyten in einem pharmazeutisch verträglichen Träger suspendiert sind.

8. In vitro-Verfahren zum Erhöhen der immunologischen Fähigkeit von Lymphozyten, umfassend das Transformieren der Lymphozyten mit einer α2-Heremans-Schmid-Glykoprotein kodierenden Nukleinsäure.

9. Verfahren nach Anspruch 8 oder Lymphozyten nach Anspruch 1, wobei die Lymphozyten zuvor aus einem Tier isoliert wurden und in transformierter Form in das gleiche Tier oder ein anderes Tier wieder einzubringen sind.

10. Lymphozyten, die mit einer α2-Heremans-Schmid-Glykoprotein kodierenden Nukleinsäure transformiert sind, wobei das α2-Heremans-Schmid-Glykoprotein TGF-β blockiert, indem es die Aktivität von Kathepsin B hemmt und die Bindung von TGF-β an den TGF-β-Rezeptor blockiert, zur Verwendung in der Behandlung eines Patienten, der unter einer Krankheit oder einem medizinischen Zustand leidet, der mit der Gegenwart von TGF-β in Zusammenhang steht, wobei die Krankheit oder der medizinische Zustand ausgewählt sind aus Krebs, Transplantation, Autoimmunkrankheiten, Wundheilung, Vernarbung und Entzündung, die ein oder mehrere Gewebe des Patienten betreffen.

11. Lymphozyten nach Anspruch 10, wobei das eine oder die mehreren Gewebe ausgewählt ist/sind aus der Gruppe bestehend aus Auge, Niere, Leber, Drüsen, sowie kardiovaskulärem, Lungen- und Herzmuskelgewebe.

12. Lymphozyten nach Anspruch 10 oder 11, wobei die Lymphozyten TGF-β durch das kompetitive Binden des α2-Heremans-Schmid-Glykoproteins an den TGF-β-Rezeptor blockieren.

13. Lymphozyten nach einem der Ansprüche 10 bis 12, wobei die Lymphozyten mittels in vivo-Gentherapie transformiert wurden.

14. Lymphozyten nach einem der Ansprüche 10 bis 12, wobei die Lymphozyten mittels ex vivo-Gentherapie transformiert wurden.

15. Lymphozyten nach einem der Ansprüche 10 bis 14, wobei die Lymphozyten in einer pharmazeutischen Zusammensetzung enthalten sind, die ferner einen pharmazeutisch akzeptablen Träger umfasst.

16. Lymphozyten nach einem der Ansprüche 10 bis 15, wobei die Nukleinsäure in einem viralen Vektor präsentiert wird.

17. Lymphozyten nach Anspruch 16, wobei der virale Vektor einen Adenovirusassoziierten viralen Vektor umfasst.

18. Isolierter Lymphozyt, der mit einer α2-Heremans-Schmid-Glykoprotein kodierenden Nukleinsäure transformiert ist.

## Revendications

1. Lymphocytes transformés par un acide nucléique codant pour la glycoprotéine α2-Heremans-Schmid pour une utilisation dans le traitement d'un patient souffrant d'un cancer.

2. Lymphocytes selon la revendication 1, où lesdits lymphocytes sont isolés à partir d'au moins un animal porteur de tumeur.

3. Lymphocytes selon la revendication 2, où ledit animal porteur de tumeur est le même que ledit patient.

4. Lymphocytes selon l'une quelconque des revendications 1 à 3, où ledit acide nucléique est introduit au moyen d'un vecteur viral adéno-associé.

5. Lymphocytes selon l'une quelconque des revendications 1 à 4, où lesdits lymphocytes sont compris dans une composition pharmaceutique qui est destinée à être administrée par injection desdits lymphocytes transformés par voie intraveineuse, intrahumorale, intramusculaire ou sous-cutanée.

6. Lymphocytes selon l'une quelconque des revendications 1 à 5, où lesdits lymphocytes sont enrichis en lymphocytes exprimant le CD8.

7. Lymphocytes selon l'une quelconque des revendications 1 à 6, où lesdits lymphocytes transformés sont suspendus dans un support pharmaceutiquement acceptable.

8. Méthode *in vitro* pour améliorer la capacité immunologique de lymphocytes comprenant la transformation desdits lymphocytes avec un acide nucléique codant pour la glycoprotéine α2-Heremans-Schmid.

9. Méthode selon la revendication 8 ou lymphocytes selon la revendication 1, où lesdits lymphocytes sont préalablement isolés à partir d'un animal et destinés à être réintroduit en une forme transformée au même animal ou à un autre animal.

10. Lymphocytes qui sont transformés par un acide nucléique codant pour la glycoprotéine α2-Heremans-Schmid, où la glycoprotéine α2-Heremans-Schmid bloque le TGF-β par inhibition de l'activité cathepsine B et blocage de la liaison du TGF-β avec le récepteur au TGF-β, pour une utilisation dans le traitement d'un patient souffrant d'une maladie ou d'un état médical associé à la présence de TGF-β, dans laquelle ladite maladie ou ledit état médical est choisi parmi le cancer, la transplantation, les troubles auto-immuns, la cicatrisation de plaie, la cicatrisation, et l'inflammation affectant un ou plusieurs tissus dudit patient.

11. Lymphocytes selon la revendication 10, où un ou plusieurs tissus est/sont choisi dans le groupe constitué de l'oeil, du rein, du foie, des glandes, du tissu cardiovasculaire, pulmonaire et myocardique.

12. Lymphocytes selon la revendication 10 ou 11, où lesdits lymphocytes bloquent le TGF-β par liaison compétitive de la glycoprotéine α2-Heremans-Schmid au récepteur au TGF-β.

13. Lymphocytes selon l'une quelconque des revendications 10 à 12, où les lymphocytes sont transformés par thérapie génique *in vivo.*

14. Lymphocytes selon l'une quelconque des revendications 10 à 12, où les lymphocytes sont transformés par thérapie génique *ex vivo.*

15. Lymphocytes selon l'une quelconque des revendications 10 à 14, où les lymphocytes sont compris dans une composition pharmaceutique qui comprend en outre un support pharmaceutiquement acceptable.

16. Lymphocytes selon l'une quelconque des revendications 10 à 15, où ledit acide nucléique est présenté dans un vecteur viral.

17. Lymphocytes selon la revendication 16, où le vecteur viral comprend un vecteur viral associé à un adénovirus.

18. Lymphocyte isolé transformé par un acide nucléique codant pour la glycoprotéine α2-Heremans-Schmid.
